# EUROPEAN PATENT APPLICATION

(11) **EP 4 636 088 A1**
(43) Date of publication of application: **22.10.2025**
(21) Application number: 23902784.0
(22) Date of filing: 14.12.2023
(51) Int. Cl.: C12N 15/54, C12N 9/12, A01H 5/00, A01H 6/82

(54) **POLYNUCLEOTIDE, PROTEIN, BIOLOGICAL MATERIAL, AND USE THEREOF IN IMPROVING QUALITY OF PLANT FRUIT**

(30) Priority: 15.12.2022 CN 202211616622
(71) Applicant: Agricultural Genomics Institute at Shenzhen, Chin. Acad. of Agricultural Sciences (Shenzhen Branch, Guangdong Lab. for Lingnan Modern Agriculture), Shenzhen, Guangdong 518124 (CN); Institute of Vegetables and Flowers, Chinese Academy of Agricultural Sciences, Beijing 100081 (CN); Agricultural Genomics Institute at Shenzhen, Chinese Academy of Agricultural Sciences, Shenzhen, Guangdong 518124 (CN)
(72) Inventor: HUANG, Sanwen, Shenzhen, Guangdong 518124 (CN); ZHANG, Jinzhe, Shenzhen, Guangdong 518124 (CN); LYU, Hongjun, Shenzhen, Guangdong 518124 (CN); CHEN, Jie, Shenzhen, Guangdong 518124 (CN); ZHU, Guangtao, Shenzhen, Guangdong 518124 (CN)
(74) Representative: Murgitroyd & Company
(86) International application number: PCT/CN2023/138699
(87) International publication number: WO 2024/125590

(57) **Abstract**

The present application belongs to the technical field of biology, and provides a polynucleotide, a protein, a biological material, and use thereof in the improvement of the quality of a plant fruit. The polynucleotide includes any one of the sequences set forth in SEQ ID NOs: 3, 11, 23-36, and 95-108; a complementary sequence, a degenerate sequence, or a homologous sequence thereof; a sequence hybridized with the sequence or a complementary sequence thereof under rigorous conditions, and a cDNA sequence of the sequence. It is found in the research that the gene is a key regulatory gene affecting the sugar content of the fruit, and in the use, the content of SSC, glucose, fructose, citric acid, and malic acid of the plant fruit can be regulated by regulating the protein function, the expression level, and the like of CDPK gene without significantly affecting the weight of the fruit, such that the quality and flavor of a tomato fruit are improved.

## Description

### FIELD OF THE INVENTION

The present disclosure relates to the field of biotechnologies, and particularly to a polynucleotide, protein, biological material and use thereof in the improvement of the quality of plant fruits.

### BACKGROUND OF THE INVENTION

Tomato, originated in the Andes region of South America, is an annual or perennial herb of *Lycopersicon* in *Solanaceae.* Tomato is the world's largest vegetable crop. In 2020, the global planting area exceeded 5 million hectares, the output was about 187 million tons, and the output value exceeded $100 billion. Soluble solids content (SSC) is an important component of tomato fruit quality, and directly affects consumer preferences and the economic value of processing tomatoes. The sugar content in tomato fruit accounts for about 55-65% of the total soluble solids, mainly including fructose and glucose. Existing studies have shown that, there is a serious negative correlation between fruit weight and sugar content. Selection of larger fruits often means the loss of high-sugar alleles, which directly leads to the generally lower SSC in modern large-fruited tomato varieties, making the sugar content of modern commercial tomato varieties basically less satisfactory. Therefore, how to create sweeter tomatoes without sacrificing fruit weight is an urgent problem that modern breeders need to solve.

Sugar accumulation in tomato fruit is a complex biological process, which is the result of the combined effects of multiple endogenous plant signals and the external environment. Key regulatory genes need to be further discovered.

### BRIEF DESCRIPTION OF THE INVENTION

In view of this, the present disclosure provides a polynucleotide, protein, biological material and use thereof in the improvement of the quality of plant fruits. The present disclosure achieves the regulation of the content of soluble solids, glucose, fructose, citric acid, and malic acid in plant fruits, as well as the regulation of fruit weight, by regulating the protein function and expression levels of CDPK and/or SuSy3, thereby improving the quality and flavor of tomato fruits.

In order to achieve the above object of the present disclosure, the present disclosure provides the following technical solutions.

The present disclosure provides a polynucleotide including at least one of the following nucleotide sequences:
(11) a sequence as set forth in any one of SEQ ID NOs: 3 and 23-36;
(12) a complementary sequence, degenerate sequence or homologous sequence of any one of the sequences set forth in SEQ ID NOs: 3 and 23-36, where the homologous sequence is a sequence having at least 75% identity to any one of the sequences set forth in SEQ ID NOs: 3 and 23-36;
(13) a sequence that hybridizes to any of the sequences set forth in SEQ ID NOs: 3 and 23-36 under stringent conditions, or a complementary sequence thereof;
(14) a cDNA sequence of any one of the sequences described in the above items (11) to (13);
(15) a sequence as set forth in any one of SEQ ID NOs: 11 and 95-108;
(16) a complementary sequence, degenerate sequence or homologous sequence of any one of the sequences set forth in SEQ ID NOs: 11, and 95-108, where the homologous sequence is a sequence having at least 75% identity to any one of the sequences set forth in SEQ ID NOs: 11 and 95-108;
(17) a sequence that hybridizes to any one of the sequences set forth in SEQ ID NOs: 11 and 95-108 under stringent conditions, or a complementary sequence thereof; and
(18) a cDNA sequence of any one of the sequences described in the above items (15) to (17).

CDPKs are a class of calcium-dependent protein kinases that regulate the expression of downstream target genes mainly through phosphorylation.

In particular embodiments provided herein, CDPK is CDPK8 and/or CDPK9. CDPK8 gene sequence is a sequence described in the above items (11)-(14); CDPK9 gene sequence is a sequence described in the above items (15)-(18).

SuSy3 is a glycosyltransferase that is widely involved in multiple metabolic processes in plants, including the transport and distribution of sucrose, starch synthesis, cellulose synthesis and cell wall composition, biotic and abiotic adversities, etc. The present disclosure discovered a calcium-dependent protein kinase 8 (SlCDPK8), which is mainly expressed in mature fruits with fixed fruit size. The present disclosure found that, CDPK8 protein can interact with SuSy3 protein to regulate the stability of SuSy3 protein by phosphorylating it, thereby affecting the sugar content of tomato fruit. In addition, the present disclosure also found that, CDPK9 also has the above functions.

In particular embodiments provided herein, CDS sequence of CDPK8 includes at least one of the following sequences:
(1) a sequence as set forth in any one of SEQ ID NOs: 1, and 37-65;
(2) a complementary sequence, degenerate sequence or homologous sequence of any one of the sequences set forth in SEQ ID NOs: 1 and 37-65, where a homologous sequence is a sequence having at least 75% identity to any one of the sequences set forth in SEQ ID NOs: 1 and 37-65;
(3) a sequence that hybridizes to any one of the sequences set forth in SEQ ID NOs: 1 and 37-65 under stringent conditions or a complementary sequence thereof; and
(4) a cDNA sequence of any one of sequences described in the above items (1) to (3).

In particular embodiments provided herein, CDS sequence of CDPK9 includes at least one of the following sequences:
(1) a sequence as set forth in any one of SEQ ID NOs: 9 and 109-152;
(2) a complementary sequence, degenerate sequence or homologous sequence of any one of the sequences set forth in SEQ ID NOs: 9 and 109-152, where a homologous sequence is a sequence having at least 75% identity to any one of the sequences set forth in SEQ ID NOs: 9 and 109-152;
(3) a sequence that hybridizes to any one of the sequences set forth in SEQ ID NOs: 9 and 109-152 under stringent conditions, or a complementary sequence thereof; and
(4) a cDNA sequence of any one of sequences described in the above items (1) to (3).

Further, a homologous sequence is a nucleotide sequence having about at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identity with original nucleotide sequence, or a corresponding cDNA molecule thereof.

In the present disclosure, a homologous sequence has at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with original nucleotide sequence and has the same function, all of which are within the protection scope of the present disclosure.

In some embodiments, a homologous sequence has 75-85%, 76-86%, 77-87%, 78-88%, 79-89%, 80-90%, 81-91%, 82-92%, 83-93%, 84-94%, 85-95%, 86-96%, 87-97%, 88-98%, 89-99%, 90-95%, 91-96%, 92-97%, 93-98%, 94-99%, 95-100%, 85-90%, 86-91%, 87-92%, 88-93% or 89-94% identity with original nucleotide sequence and has the same function, all of which are within the protection scope of the present disclosure.

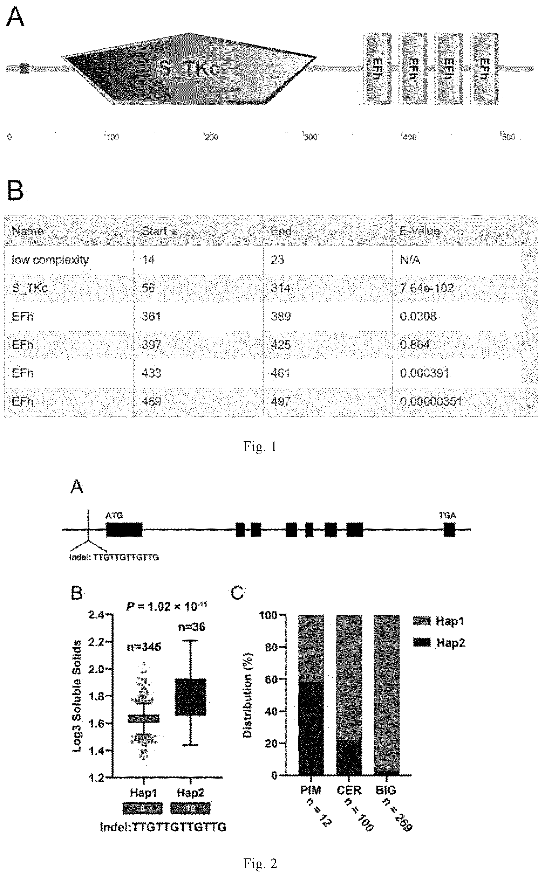

Furthermore, the homologous sequence encodes exactly the same protein conserved domain as the original nucleotide sequence. Particularly, the conserved structural domain of the protein is shown in Fig. 1.

Particularly, a nucleotide sequence molecule may be DNA such as cDNA, genomic DNA or recombinant DNA; a nucleotide sequence molecule may also be RNA, such as mRNA or hnRNA.

The nucleotide sequences of CDPK8 and/or CDPK9 are not necessarily completely the same in different tomato materials. The present disclosure provides nucleotide sequences of CDPK8 and/or CDPK9 in other tomato materials obtained during the research process, where the genome identity of CDPK8 among different varieties is 98.734-100%, and the CDS identity is 92.83-100% (see Table 1 for details), where the sequences that are not completely the same as SEQ ID NO: 3 are set forth in SEQ ID NOs: 23-36, and the sequences that are not completely the same as SEQ ID NO: 1 are set forth in SEQ ID NOs: 37-65; the genome identity of CDPK9 among different varieties is 98.734-100%, and the CDS identity is 90.59-100% (see Table 2 for details), where the sequences that are not completely the same as SEQ ID NO: 11 are set forth in SEQ ID NOs: 95-108, and the sequences that are not completely the same as SEQ ID NO: 9 are set forth in SEQ ID NOs: 109-152.

The present disclosure further provides a protein, which is at least one of the following sequences:
(21) a sequence as set forth in any one of SEQ ID NOs: 2 and 66-94;
(22) a fusion protein obtained by connecting a tag to N-terminus and/or C-terminus of any of the sequences set forth in SEQ ID NOs: 2 and 66-94;
(23) a protein having the same function obtained by substituting and/or deleting and/or adding one or more amino acid residues in any of the sequences set forth in SEQ ID NOs: 2 and 66-94;
(24) a protein having at least 75% identity to any one of the sequences set forth in SEQ ID NOs: 2 and 66-94 and having the same function;
(25) any one of the sequences set forth in SEQ ID NOs: 10 and 153-196;
(26) a fusion protein obtained by connecting a tag to the N-terminus and/or C-terminus of any of the sequences set forth in SEQ ID NOs: 10 and 153-196;
(27) a protein having the same function obtained by substituting and/or deleting and/or adding one or more amino acid residues in any of the sequences set forth in SEQ ID NOs: 10 and 153-196; and
(28) a protein having at least 75% identity with any one of the sequences set forth in SEQ ID NOs: 10 and 153-196 and having the same function.

In particular embodiments provided herein, CDPK is CDPK8 and/or CDPK9. The CDPK8 protein sequence is a sequence described in the above items (21)-(24); the CDPK9 protein sequence is a sequence described in the above items (25)-(28).

Particularly, a protein with the same function obtained by substitution and/or deletion and/or addition of one or several amino acid residues refers to a protein with exactly the same protein conserved domain. More particularly, the conserved domain of the protein is shown in Fig. 1.

Furthermore, a homologous sequence may also be a protein sequence having about at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity with original protein sequence.

In the present disclosure, a homologous sequence may also have at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with original protein sequence and has the same function, all of which are within the protection scope of the present disclosure.

In some embodiments, the homologous sequence may also have 75-85%, 76-86%, 77-87%, 78-88%, 79-89%, 80-90%, 81-91%, 82-92%, 83-93%, 84-94%, 85-95%, 86-96%, 87-97%, 88-98%, 89-99%, 90-95%, 91-96%, 92-97%, 93-98%, 94-99%, 95-100%, 85-90%, 86-91%, 87-92%, 88-93% or 89-94% identity with original protein sequence and has the same function, all of which are within the protection scope of the present disclosure.

Furthermore, the homologous sequence has exactly the same protein conserved domain as original protein sequence. Particularly, the conserved structural domain of the protein is shown in Fig. 1.

The protein sequences of CDPK8 and/or CDPK9 are not necessarily completely the same in different tomato materials. The present disclosure provides protein sequences of CDPK8 and/or CDPK9 in other tomato materials obtained during the research process, where the identity of the CDPK8 protein sequences between different varieties is 93.007-100% (see Table 1 for details), where the sequences that are not completely the same as SEQ ID NO: 2 are set forth in SEQ ID NOs: 66-94; the identity of the CDPK9 protein sequences between different varieties is 90.037-100% (see Table 2 for details), where the sequences that are not completely the same as SEQ ID NO: 10 are set forth in SEQ ID NOs: 153-196.

The present disclosure further provides a biomaterial, which is any one of the following (31) to (35):
(31) a knockout cassette for knocking out the aforementioned polynucleotide;
(32) a knockout vector for knocking out the aforementioned polynucleotide;
(33) a recombinant microorganism for knocking out the aforementioned polynucleotide;
(34) a plant cell line for knocking out the aforementioned polynucleotide; and
(35) a plant protoplast, cell or callus tissue in which any one of the above items (31) to (33) is introduced.

The present disclosure further provides use of any one of the above polynucleotide, protein and biological material in the improvement of the quality of plant fruits.

In the embodiments provided herein, the plant is a plant with edible parts such as fruits, roots, tubers, etc.

Preferably, the plants are berry plants, *Solanaceae* plants, *Rosaceae* plants, *Rutaceae* plants, *Convolvulaceae* plants, *Dioscorea* plants, *Cucurbitaceae* plants, etc., but are not limited to these plant species. As long as the plant species are recognized by those skilled in the art, they are within the protection scope of the present disclosure.

In the embodiments provided herein, berry plants include but are not limited to: tomato, grape, kiwi, papaya, pomegranate, ginseng fruit, blueberry, persimmon, passion fruit, dragon fruit, wax apple, and the like.

In the embodiments provided herein, *Solanaceae* plants include but are not limited to: potato, eggplant, tomato, pepper, wolfberry, and the like.

In the embodiments provided herein, *Rosaceae* plants include but are not limited to: pear, apple, peach, apricot, cherry, plum, prune, crabapple, loquat, hawthorn, strawberry, raspberry and the like.

In the embodiments provided herein, *Rutaceae* plants include but are not limited to: grapefruit, phellodendron, lemon, orange, mandarin orange, tangerine, and the like.

In particular embodiments provided herein, the plant is tomato.

In particular embodiments provided herein, the improvement of the quality of plant fruits includes increasing the content of soluble solids, sugar, and citric acid in the plant fruits, reducing the content of malic acid, and maintaining the weight of the fruits.

In particular embodiments provided herein, the plant is tomato, and the contents of soluble solids, sugar, citric acid, malic acid, and the weight of the tomato fruit are measured at the red ripe stage of the fruit.

In the embodiments provided herein, the sugar includes one or more of glucose, fructose, and sucrose.

In particular embodiments provided herein, the sugar includes glucose and/or fructose.

In some embodiments, the content of soluble solids, sugar or citric acid in the fruit of the plant of interest is at least 30% higher than that of the recipient plant. In some embodiments, the content of soluble solids, sugar or citric acid in the fruit of the plant of interest is at least 10%, at least 20%, at least 30%, at least 40%, at least 50%, at least 60%, at least 70%, at least 80%, at least 90%, or at least 100% higher than that of the recipient plant.

In some embodiments, the malic acid content in the fruit of the plant of interest is at least 30% lower than that of the recipient plant. In some embodiments, the malic acid content in the plant fruit of the target plant is at least 10%, at least 20%, at least 30%, at least 40%, at least 50%, at least 60%, at least 70%, at least 80%, at least 90%, or at least 100% lower than that of the recipient plant.

The present disclosure further provides use of a polynucleotide, protein, and biological material in the improvement of the quality of a plant fruit, where the phosphorylation level of SuSy3 protein is reduced by at least one of the following methods:
(1) inhibiting the expression of CDPK or inactivating the phosphorylase of CDPK; the polynucleotide of CDPK gene is the aforementioned polynucleotide, or CDPK protein is the aforementioned protein;
(2) mutating one or more amino acids in SuSy3 protein, where the mutation site is a phosphorylation site mediated by CDPK, without altering the glycosyltransferase activity of the SuSy3 protein;
(3) specifically overexpressing the SuSy3 protein in the fruit.

Particularly, the present disclosure can control the phosphorylation level of SuSy3 protein and the quality of tomato fruit and improve the flavor of tomato fruit by the following three methods.

### 1. Functional Regulation of CDPK

The present disclosure first discovered that, CDPK8/CDPK9-SuSy3 interaction regulates sugar accumulation in tomato fruit. The functional loss of CDPK8 and CDPK9 can promote the accumulation of glucose and fructose in tomato fruit, and increase SSC. This gene function is reported for the first time in tomatoes, and this gene is an important gene for sugar accumulation in tomatoes. The protein stability of SuSy3 protein is regulated by CDPK8 and CDPK9, and is also involved in the biological process of tomato sugar accumulation. In present disclosure, the sugar content and SSC of tomatoes and the weight of fruit can be controlled by regulating the protein functions and expression levels of CDPK8, CDPK9, and SuSy3.

The present disclosure identified two key genes CDPK8 and CDPK9 that could regulate SSC of tomato fruit, and utilized gene editing and other technologies to regulate the gene expression and protein function of CDPK8 and CDPK9 by inserting a 12bp sequence in the promoter region and mutating the key kinase domain in the coding region, thereby creating a new tomato breeding material with increased SSC, glucose, fructose, and citric acid contents in the fruit, decreased malic acid content, and no significant change in single fruit weight.

By regulating the function of CDPK8, when CDPK8 function is lost, SuSy3 protein is not phosphorylated, and the stability of the protein can be maintained to function normally in the sugar synthesis pathway. Therefore, sweet tomatoes can be created without sacrificing fruit weight, and their glucose and fructose content can be increased by 30%.

CDPK9 and CDPK8 are homologous genes, and CDPK9 protein can also interact with SuSy3 protein to phosphorylate SuSy3. When CDPK8 and its homologous gene CDPK9 mutate at the same time, an additive effect can be produced, further expanding the impact of CDPK8 on sugar content and fruit sweetness.

### 2. Mutation of SuSy3

CDPK can phosphorylate the serine at position 11 (S11) of SuSy3 protein. When the serine at position 11 mutates into other amino acid, it can prevent CDPK8 from phosphorylating SuSy3. SuSy3 protein is not phosphorylated, which maintains the stability of the protein and allows it to function normally in the sugar synthesis pathway, thus creating sweet tomatoes without sacrificing fruit weight.

### 3. Specific Overexpression of SuSy3 Protein in Fruit

The present disclosure inserts a promoter E8 for specific expression in tomato fruits, thereby causing the SuSy3 protein to be specifically overexpressed in the fruit. Even though CDPK can phosphorylate part of the SuSy3 protein and cause degradation, a large amount of SuSy3 protein with normal function still exists in the tomato fruit, thereby playing a normal role in the sugar synthesis pathway. Therefore, sweet tomatoes can be created without sacrificing the weight of the fruit.

In particular embodiments provided herein, the genome sequence of SuSy3 includes at least one of the following sequences:
(1) a sequence as set forth in SEQ ID NO: 19;
(2) a complementary sequence, degenerate sequence or homologous sequence of the sequence set forth in SEQ ID NO: 19, where a homologous sequence is a sequence having at least 75% identity with SEQ ID NO: 19;
(3) a sequence that hybridizes to the sequence set forth in SEQ ID NO: 19 under stringent conditions or a complementary sequence thereof; and
(4) a cDNA sequence of any one of sequences in the above items (1) to (3).

In particular embodiments provided herein, CDS sequence of SuSy3 includes at least one of the following sequences:
(1) a sequence as set forth in SEQ ID NO: 17;
(2) a complementary sequence, degenerate sequence or homologous sequence of the sequence set forth in SEQ ID NO: 17, where a homologous sequence is a sequence having at least 75% identity with SEQ ID NO: 17;
(3) a sequence that hybridizes to the sequence set forth in SEQ ID NO: 17 under stringent conditions or a complementary sequence thereof; and
(4) a cDNA sequence of any one of sequences in the above items (1) to (3).

In particular embodiments provided herein, the protein sequence of SuSy3 includes at least one of the following sequences:
(1) a sequence as set forth in SEQ ID NO: 18;
(2) a fusion protein obtained by connecting a tag to N-terminus and/or C-terminus of the sequence set forth in SEQ ID NO: 18;
(3) a protein having the same function obtained by substituting and/or deleting and/or adding one or more amino acid residues in the sequence set forth in SEQ ID NO: 18; and
(4) a protein having at least 75% identity with the sequence set forth in SEQ ID NO: 18 and having the same function.

In particular embodiments provided herein, in the above first method, the method for inhibiting the expression of CDPK or inactivating the phosphorylase of CDPK includes regulating a promoter and/or changing a coding region.

In particular embodiments provided herein, the method for inhibiting the expression of CDPK or inactivating the phosphorylase of CDPK is to regulate the promoter, and the specific method for regulating the promoter is: inserting the sequence as set forth in SEQ ID NO: 22 into the CDPK promoter region, for example, inserting into the CAACA conserved site region. However, the insertion sequence is not limited thereto, as long as it can achieve the technical effect of inhibiting CDPK expression.

Through analysis of the CDPK8 genome sequence, it was found that there is a 12bp sequence variation in the promoter region (TTGTTGTTGTTG, as set forth in SEQ ID NO: 22), which is closely related to the SSC of tomato fruit. The Indel sequence can inhibit gene expression by recruiting the RAV transcription repressor. The research results showed that, SSC of tomato materials containing the 12bp Indel sequence is significantly higher than that of tomato materials lacking the 12bp Indel sequence, indicating that the 12bp Indel sequence variation is a key site for regulating the SSC of tomato fruit.

In particular embodiments provided herein, the method for inhibiting the expression of CDPK or inactivating the phosphorylase of CDPK is to change the coding region, and changing the coding region particularly includes: deleting, inserting and/or mutating (non-synonymous mutation) a key kinase domain of the coding region.

In the particular embodiments provided herein, changing the coding region particularly includes: deleting an ATP binding domain, or changing the coding region to terminate protein translation prematurely.

In particular embodiments provided herein, the key kinase domain of the coding region includes leucine at position 62 (L62) and glycine at position 63 (G63) of CDPK8, and leucine at position 64 (L64) and glycine at position 65 (G65) of CDPK9. However, the key functional domain is not limited thereto, as long as the technical effect of inhibiting CDPK expression can be achieved.

Particularly, the full length of CDPK8 contains 533 amino acids. The results of this study showed that, the leucine at position 62 (L62) and the glycine at position 63 (G63) of CDPK8 are essential for the catalytic activity of CDPK8 phosphorylase. When L62 and G63 are missing, the activity of CDPK8 phosphorylase will be lost through the loss of ATP binding domain or premature termination of protein translation, thus it will be unable to phosphorylate SuSy3 protein.

Particularly, the full length of CDPK9 contains 533 amino acids. The results of this study showed that, the leucine at position 64 (L64) and the glycine at position 65 (G65) of CDPK9 are essential for the catalytic activity of CDPK9 phosphatase. When L64 and G65 are missing, the activity of CDPK9 phosphatase will be lost through the loss of ATP binding domain or premature termination of protein translation, thus it will be unable to phosphorylate SuSy3 protein.

In particular embodiments provided herein, the method for inhibiting the expression of CDPK or inactivating the phosphorylase of CDPK includes at least one of: CRISPR/Cas9 gene editing technology, promoter editing technology, TALEN technology, T-DNA insertion, EMS mutagenesis, or ZFN technology.

In particular embodiments provided herein, in the above second method, the mutation site is serine at position 11 of the SuSy3 protein.

In particular embodiments provided herein, in the above second method, the serine at position 11 of the SuSy3 protein is mutated to alanine.

In particular embodiments provided herein, in the above third method, the promoter for specifically overexpressing the SuSy3 protein in the fruit is the E8 promoter.

In the embodiments provided herein, the tomato varieties include but are not limited to: SV7845TH, SVTH4018, Antelaisi, SV4224TH, SV8795TG, SV3026TG, Meishuai, SV0313TG, Hongsui, Diliya, Deruisi, Fengege, Oushuo, Huifu, SVTH1366, Ouke, Meibei, BOLZANO, DRC 564, DRK 936, TOMIMARU MUCHOO), DELISHER, Strabena, DR0607TC, Anbeisi, Jiuli, Camry, Winter warm, Sibeide, Kevin, Jiuli 200, Tiannaxi No. 3, Ruifei No. 5, Ruifei No. 2, Ameizi No. 5, PF207008, Juli, Beiying, Zidali, Ruifei, Kaisa, Ono, Fenbeiying, Rabi, 72-CH0353, Fengshou 74-560, Zulfia (73-610), Florantino (72-163), Fulltom (72-729), Forticia (72-719), Glorioso (74-112), Cappricia (72-466), Brioso (72-130), Kawaguchi (72-541), Kivu (72-629), Tatami (72-175), Tarffy 6917 (76-IM6917), Genery (72-192), Moscatel (74-104), Siberite 915 (73-915), Siberite 916 (73-916), Plumola (72-001), Messina (73-47), Paulanca (72-534), Logure (73-571), Santina (72-763), Operino (72-187), Ternetto (72-190), Tomary (72-191), Ruifen 882 (73-882), TY 12, Beril (73-14), Abellus (73-583), Fortesa (72-152), Lidertom (74-254), Solarino (72-150), Vacetto (72-164), Reddery (72-008), Endeavour (72-487), Suncery (74-132), Gourami (72-021), Betty (72-126) Pareso, Hibachi (72-241), Aruru (72-193), Lauster, Magino (72-195 ), Yingla, Gutian, Solana, Andara, Andolina, Keren, Summer Sunshine, Honglilai, Diruisen, Qiulian, Nicola, Saint Laurent, Fendi, Dali, Hanyu 522, Ainila, Babaoli, Lasaier, Jinlong, Walter, Kailong, Dorothy, Fulaixin, Raphael, Haiyue, Luola, Annecy, Rila, Sidi, Fola 3661, Shiluoqi, Davidson, Jinpeng No. 8, Jinpeng 101, Jinpeng 950, Jinpeng 2095, Jinpeng Sengbiya 45, Jinpeng Sengbiya 50, Jinpeng No. 8 B, Jinpeng No. 9, Jinpeng No. 10, Jinpeng 148, Jinpeng 152, Jinpeng 218, Jinpeng 261, Jinpeng 322, Jinpeng 945, Jinpeng 951, Jinpeng 1186, Jinpeng 1198, Jinpeng Qiusheng, Jinpeng Rongwei, Jinpeng Tianyi No. 2, Jinpeng 102, Jinpeng 1828, Jinpeng M708, Jinpeng M5038, Jinpeng No. 11, Jinpeng 12-2, Jinpeng 18-98, Jinpeng 236, Jinpeng 1729, Jinpeng Black Marshal, Jinpeng Sengbiya 04, Jinpeng 11-21, Jinpeng 1521, Jinpeng 1605, Jinpeng M6, Jinpeng M7, Jinpeng M158, Jinpeng No. 5, Jinpeng Xianai, Jinpeng No. 1, Jinpeng 128, Jinpeng M215, Jinpeng 513, Jinpeng 515, Jinpeng No. 6, Jinpeng No. 3, Jinpeng Red String 132, Jinpeng Pink Girl No. 2, Jinpeng Little Yellow Crown, Jinpeng Pink Girl No. 1, Jinpeng Little Yellow Crown 58, Jinpeng No. 19, Jinpeng No. 44, Jinpeng No. 53, Jinpeng No. 75, Jinpeng 151, Jinpeng Show Girl, Holy Banquet 1523, Xilaide No. 1, Darwin, Bora, Ferrari, Osina, Olina, Nunhem 1618, Nunhem 1718, Praise No. 2, Holy Banquet 3767, Blooming Red 6415, Jin Xiaoling, Pink Girl, Pink Girl No. 2, Lifei, Pink Taro No. 3, Delicious 925, Purple Taro, Athena, Show Taro, Qing Taro, Pink Lide, Pink Lide No. 2, Red Rise, Red Rise No. 2, Beautiful 601, Yellow Crown 16, Yellow Crown No. 2, Yellow Crown No. 3, Golden Pear, Yellow Little Y, Black Pearl No. 2, Guyu Tianfei^{®}F1, Guyu Tianfei^{®} No. 2, Guyu Tianfei^{®} No. 3, Guyu Tianfei^{®} No. 6 F1, Guyu Tianfei^{®} No. 7, Guyu Tianfei^{®} No. 8, Guyu Tianfei^{®} No. 9, Guyu Tianfei^{®} No. 10, Guyu Tianfei^{®} No. 11, Guyu zhuofen^{®}227, Guyu zhuofen^{®} No. 1, Guyu zhuofen^{®} No. 2, Guyu zhuofen^{®} No. 3, Guyu zhuofen^{®} No. 5, Guyu z Tianci No. 1, Guyu z Tianci No. 2, Guyu z Tianci No. 3, Guyu z Tianci No. 4, Guyu z Tianci No. 5, Guyu z Tianci No. 6, Guyu Tianci 595, Guyu Tianci 585, Guyu Tianci 575, Guyu Tianci 565F1, Guyu Tianqi No. 1, Guyu Tianqi No. 2, Guyu Tianqi No. 3, Guyu zhuohang^{®} No. 1, Guyu zhuohang^{®} No. 2, Guyu zhuohang^{®} No. 3, Guyu zhuohang^{®} No. 4, Red Kerry No. 1, Guyu Tianmi No. 1, Guyu Tianmi No. 2, Guyu Tianmi No. 3, Hehong No. 1 Tomato, Hehong No. 2 Tomato, Hehong No. 3 Tomato, Yingjiahe No. 3, Yingjiahe No. 2, Yingjiahe No. 5, Yingjiahe No. 4, Yingjiahe No. 1, Pengbo No. 3, Pengbo No. 2, Pengbo No. 1, Herunsheng No. 4, Herunsheng No. 3, Herunsheng No. 2, Herunsheng No. 1, Baoliyuan No. 6, Baoliyuan No. 5, Baoliyuan No. 4, Baoliyuan No. 3, Baoliyuan No. 2, Baoliyuan No. 1, Millennium, Fengzhu, Xiaoxia, Chuntao, Guanghui 101, Money maker, M82, Ailsa Craig, Provence, Ruixing Dabao, Pink Baby, Zhongshu No. 4, Zhongshu No. 5, Busan 88, Pink Taro, etc.

In some embodiments, the tomato is selected from the group consisting of: Money maker, M82, Ailsa Craig, Millennium, Provence, Ruixing Dabao, Zidali, Pink Baby, Summer Sunshine, Luola, Zhongshu No. 4, Zhongshu No. 5, Busan 88, Pink Taro, Antelaisi, Rabi, Lauster, Jinpeng No. 8, Jinpeng Little Yellow Crown, Guyu Tianci^{®} No. 2, and Chuntao.

The present disclosure further provides a plant or a related product thereof, where the plant is one of the following plants:
(1) a plant obtained by using the aforementioned biological material, or a plant obtained by using the aforementioned method;
(2) a progeny formed by self-pollination of the plant described in the above item (1), and a plant grown from the progeny; and
(3) a progeny formed by hybridization of the plant described in the above item (1) with other varieties, and a plant grown from the progeny.

In particular embodiments provided herein, the related product is a plant part, plant cell, tissue culture, protoplast, fruit, pollen or seed.

The present disclosure further provides a commercial plant product, which is a commercial plant product made from the above plant or a related product thereof; the commercial plant product includes food, medicine, cosmetics/skin care product, feed or other products.

In the particular embodiments provided herein, the food includes: fresh fruit, dried fruit, frozen fruit, pickled fruit, candied fruit, fried fruit or various processed forms of fruit; the various processed forms of fruit include fruit strip, fruit block, fruit slice, fruit particle, fruit powder, fruit paste, fruit juice, or fermented product.

The medicine includes: various dosage forms made from medicinal parts of the plant.

The cosmetics/skin care product includes: facial cleanser, toner, lotion, face cream, essence, facial mask, eye cream, eye mask, primer lotion, sunscreen, liquid foundation, or BB cream.

The feed includes: liquid feed, solid feed, semi-solid feed, or feed raw material.

The present disclosure further provides a method for producing a commercial plant product, which includes obtaining the above plant or a related product thereof to produce the commercial plant product.

The present disclosure further provides a tomato plant or seed capable of producing tomato fruit at mature, the tomato fruit having an average total soluble solids content (SSC) of at least 4.0 and an average fresh fruit weight of at least 80g.

The present disclosure further provides a tomato plant or seed capable of producing tomato fruit at maturity, the tomato fruit having an average glucose level of at least 10 mg/g FW and an average fresh fruit weight of at least 80g.

The present disclosure further provides a tomato plant or seed capable of producing tomato fruit at maturity, the tomato fruit having an average fructose level of at least 10 mg/g FW and an average fresh fruit weight of at least 80g.

Preferably, the tomato plant or seed has a genetic background substantially derived from any one of the above-mentioned tomato varieties.

The present disclosure further provides an improved tomato plant or seed including one or more genetic modifications and is capable of producing tomato fruits at maturity, and it includes:
(i) an average total soluble solids content (SSC) which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
where both the control tomato plant or seed and the modified tomato plant or seed include substantially the same genetic background, except for the one or more genetic modifications.

The present disclosure further provides an improved tomato plant or seed, where it includes one or more genetic modifications and is capable of producing tomato fruits at maturity, and it includes:
(i) an average glucose level which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
where both the control tomato plant or seed and the modified tomato plant or seed include substantially the same genetic background, except for the one or more genetic modifications.

The present disclosure further provides an improved tomato plant or seed, where it includes one or more genetic modifications and is capable of producing tomato fruits at maturity, and it includes:
(i) an average fructose level which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
where both the control tomato plant or seed and the modified tomato plant or seed include substantially the same genetic background, except for the one or more genetic modifications.

Preferably, the one or more genetic modifications are located in a gene selected from the group consisting of: CDPK8, CDPK9, SuSy3, and a homolog thereof.

Preferably, the one or more genetic modifications regulate the expression or activity of one or more genes selected from the group consisting of: CDPK8, CDPK9, SuSy3, and a homolog thereof.

Preferably, the one or more genetic modifications include a transgene targeting a gene selected from the group consisting of: CDPK8, CDPK9, SuSy3, and a homologue thereof.

In particular embodiments provided herein, the one or more genetic modifications are in a gene of any one of the sequences set forth in SEQ ID NOs: 3, 11, 23-36, and 95-108.

In particular embodiments provided herein, the one or more genetic modifications result in the prevention or regulation of phosphorylation of SuSy3 or a homologue thereof.

In particular embodiments provided herein, the one or more genetic modifications result in the prevention or regulation of serine phosphorylation at position 11 amino acid residue of SuSy3.

In particular embodiments provided herein, the one or more genetic modifications result in delayed degradation of SuSy3 or a homolog thereof.

In particular embodiments provided herein, the one or more genetic modifications result in over-accumulation of SuSy3 or a homolog thereof.

In particular embodiments provided herein, the one or more genetic modifications include CDPK8 mutation, CDPK9 mutation, and double mutation of CDPK8 and CDPK9.

In particular embodiments provided herein, the one or more genetic modifications include a CDPK8 deletion mutation, a CDPK9 deletion mutation, and a double deletion mutation of CDPK8 and CDPK9.

In particular embodiments provided herein, the one or more genetic modifications include a 12 bp (a sequence set forth in SEQ ID NO: 22) insertion in the CDPK8 promoter present in Hap2.

The present disclosure further provides a breeding method for improving plant fruit quality, where it includes using a molecular marker to assist in plant breeding, where the molecular marker is a molecular marker for detecting presence or absence of CDPK8 and/or CDPK9.

This disclosure detected different tomato materials and found that, there are multiple mutation sites in high-sugar tomato varieties (as shown in the table below). These mutation sites are closely linked to the above-mentioned 12bp key mutation site, and can be used to screen for high-sugar content phenotypes.

In particular embodiments provided herein, the molecular markers for detecting presence or absence of CDPK8 include at least one of the following:

| S12.50 ch11 | Reference | Alternative | Region | Position relative to the starting codon ATG | Detail |
|---|---|---|---|---|---|
| 51186147 | A | C | Downstream | +9460bp | Most significant mutation |
| 51188895 | G | C | Downstream | +6712bp | Mutation |
| 51188914 | A | G | Downstream | +6693bp | Mutation |
| 51189060 | C | T | Downstream | +6547bp | Mutation |
| 51189072 | T | C | Downstream | +6535bp | Mutation |
| 51189139 | G | A | Downstream | +6468bp | Mutation |
| 51189300 | G | T | Downstream | +6307bp | Mutation |
| 51189382 | C | T | Downstream | +6225bp | Mutation |
| 51189503 | C | T | Downstream | +6104bp | Mutation |
| 51189521 | G | A | Downstream | +6086bp | Mutation |
| 51189733 | A | - | Downstream | +5874bp | Deletion |
| 51189896 | C | G | Downstream | +5711bp | Mutation |
| 51189975 | - | T | Downstream | +5632bp | Insertion |
| 51190011-51190014 | CATT | C--T/CA-/TA-T | Downstream | +5596bp/+5593bp | Deletion/Mutation |
| 51190111 | A | - | Downstream | +5496bp | Deletion |
| 51190132 | T | C | Downstream | +5475bp | Mutation |
| 51190458 | G | A | Downstream | +5149bp | Mutation |
| 51190546 | G | A | Downstream | +5061bp | Mutation |
| 51191042 | C | T | Intron | +4565bp | Mutation |
| 51191171 | G | A | Intron | +4436bp | Mutation |
| 51191427-51191440 | AAGTCCCAAGC CCC | ---------- | Intron | +4180bp/+4167bp | Deletion |
| 51191864 | G | A | Intron | +3743bp | Mutation |
| 51191926 | A | G | Intron | +3681bp | Mutation |
| 51192112 | A | G | Exon | +3495bp | Synonymous mutation |
| 51192245 | A | C | Intron | +3362bp | Mutation |
| 51193364 | C | A | Intron | +2243bp | Mutation |
| 51193567 | G | C | Exon | +2040bp | Synonymous mutation |
| 51193714 | G | A | Exon | +1893bp | Non-synonymous mutation T194I |
| 51194369 | T | A | Intron | +1238bp | Mutation |
| 51194756 | G | A | Intron | +851bp | Mutation |
| 51194964 | C | T | Intron | +643bp | Mutation |
| 51195083 | T | C | Intron | +524bp | Mutation |
| 51195344 | T | C | Exon | +263bp | Synonymous mutation |
| 51195539 | A | C | Exon | +68bp | Synonymous mutation |
| 51195552-51195554 | TTC | --- | Exon | +55bp/+53bp | In-frame deletion mutation K18- |
| 51195789 | T | - | Promoter | -182bp | Deletion |
| 51195816 | - | A | Promoter | -209bp | Insertion |
| 51195897 | G | A | Promoter | -290bp | Mutation |
| 51196183 | C | A | Promoter | -576bp | Mutation |
| 51196264 | T | - | Promoter | -657bp | Deletion |
| 51196441 | G | A | Promoter | -834bp | Mutation |
| 51196462 | G | A | Promoter | -855bp | Mutation |
| 51196483 | T | C | Promoter | -876bp | Mutation |
| 51196878-51196880 | ATG | - | Promoter | -1271bp/-1273bp | Deletion |
| 51196906 | T | C | Promoter | -1299bp | Mutation |
| 51196934 | C | G | Promoter | -1327bp | Mutation |
| 51196967 | G | A | Promoter | -1360bp | Mutation |
| 51197003 | G | C | Promoter | -1396bp | Mutation |
| 51197075 | T | G | Promoter | -1468bp | Mutation |
| 51197085 | G | A | Promoter | -1478bp | Mutation |
| 51197106 | A | T | Promoter | -1499bp | Mutation |
| 51197121 | T | C | Promoter | -1514bp | Mutation |
| 51197214 | A | G | Promoter | -1607bp | Mutation |
| 51197221 | G | C | Promoter | -1614bp | Mutation |
| 51197324 | C | T | Promoter | -1717bp | Mutation |
| 51197357 | T | - | Promoter | -1750bp | Deletion |
| 51197369 | G | A | Promoter | -1762bp | Mutation |
| 51197378 | G | T | Promoter | -1771bp | Mutation |
| 51197397 | T | A | Promoter | -1790bp | Mutation |
| 51197421 | C | A | Promoter | -1814bp | Mutation |
| 51197480 | G | A | Promoter | -1873bp | Mutation |
| 51197604 | C | T | Promoter | -1997bp | Mutation |
| 51197708 | - | AA | Promoter | -2101bp | Insertion |
| 51197738 | G | A | Promoter | -2131bp | Mutation |

The present disclosure further provides a gene fragment set forth in SEQ ID NO: 22, which can be inserted into the CDPK8 gene to inhibit the expression of the CDPK8 gene.

In particular embodiments provided herein, the insertion site of the gene fragment set forth in SEQ ID NO: 22 is the promoter region of the CDPK gene on chromosome 11 of tomato.

The present disclosure further provides a class of CDPK genes, where the gene ID of the CDPK gene is selected from the group consisting of: Solyc01g006730, Solyc01g006840, Solyc01g008740, Solyc01g112250, Solyc02g032820, Solyc02g083850, Solyc03g031670, Solyc03g033540, Solyc03g113390, Solyc04g009800, Solyc04g049160, Solyc04g081910, Solyc05g056570, Solyc06g065380, Solyc06g073350, Solyc07g064610, Solyc08g008170, Solyc09g005550, Solyc10g074570, Solyc10g076900, Solyc10g079130, Solyc10g081640, Solyc10g081740, Solyc11g006370, Solyc11g018610, Solyc11g064900, and Solyc12g099790.

Compared with the conventional technology, the present disclosure has the following beneficial effects:

The present disclosure found that, CDPK8, CDPK9 and SuSy3 are key regulatory genes that affect the sugar content of fruits. In application, the protein functions and expression levels of CDPK8, CDPK9 and SuSy3 can be regulated to achieve the regulation of SSC, glucose, fructose, citric acid and malic acid content in plant fruits without significantly affecting the fruit weight, thereby improving the quality and flavor of tomato fruit.

### BRIEF DESCRIPTION OF THE DRAWINGS

Fig. 1 shows the protein sequence information of tomato CDPK8 gene; in which Fig. A shows the prediction of the conserved domain of the protein sequence of tomato CDPK8 gene, and Fig. B shows the detailed information of the position of the conserved domain of tomato CDPK8 protein sequence; name indicates the name of the conserved domain, start indicates the starting position of the conserved domain, end indicates the ending position of the conserved domain, and E-value indicates the expected value.
Fig. 2 shows the tomato CDPK8 genome sequence information and typing information of natural population; in which Fig. A shows the tomato CDPK8 genome sequence information, Fig. B shows that a 12bp sequence variation can divide the natural tomato population into two haplotypes, and Fig. C shows the proportion of tomato materials with the Hap2 haplotype in *Solanum pimpinellifolium* (PIM), *Lycopersicon esculentum var. cerasiforme* A.Gray (CER) and BIG tomato (BIG) populations.

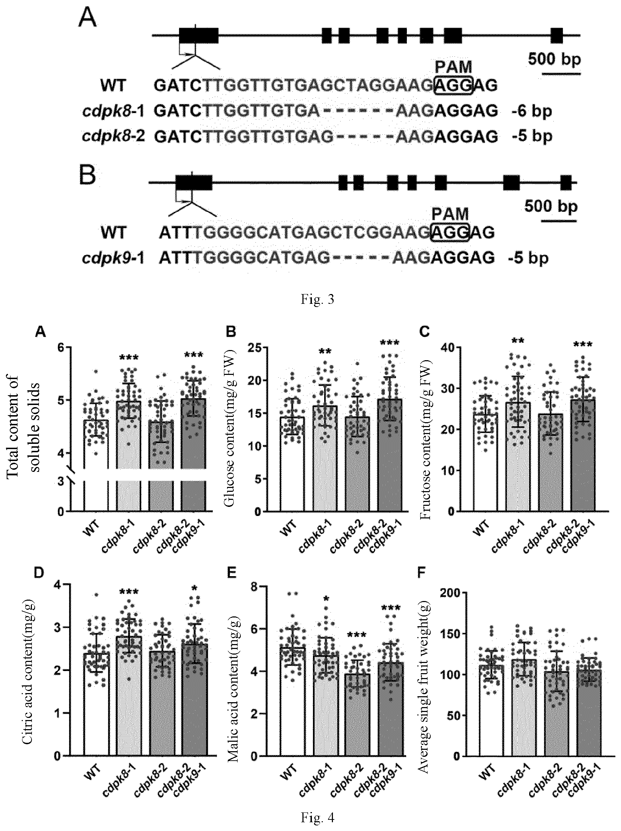

Fig. 3 shows homozygous mutant strains of tomato CDPK genes; in which Fig. A shows cdpk8-1 genotype 1 and cdpk8-2 genotype of homozygous mutant strains of tomato CDPK8 gene; and Fig. B shows cdpk9-1 genotype of homozygous mutant strains of tomato CDPK9 gene.
Fig. 4 shows the results of phenotypic identification of homozygous mutant strains of tomato CDPK8/CDPK9 genes.

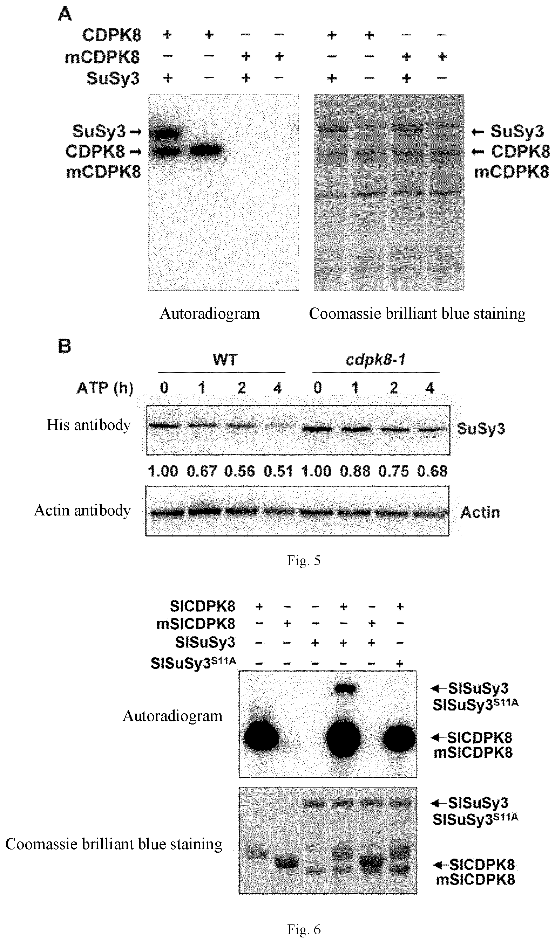

Fig. 5 shows the interaction between tomato CDPK8 protein and sucrose synthase SuSy3 protein and its effect on the stability of SuSy3 protein; in which Fig. A shows that tomato CDPK8 protein has phosphokinase activity, and can phosphorylate sucrose synthase SuSy3 protein; Fig. B shows the stability of SuSy3 protein.
Fig. 6 shows the key sites for phosphorylation of SuSy3 protein by CDPK8.

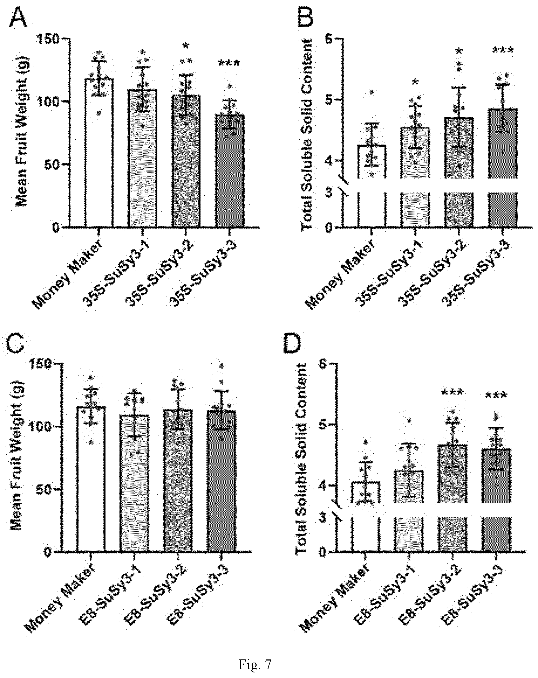

Fig. 7 shows the phenotypic identification results of SuSy3 overexpression transgenic lines (35S-SuSy3-1/2/3) and fruit-specific expression transgenic lines (E8-SuSy3-1/2/3); mean fruit weight represents the average fruit weight, and total soluble solid content represents the total soluble solid content.

Particularly, in Figs. 4 and 7: * indicates P-Value < 0.05; ** indicates P-Value < 0.01; *** indicates P-Value < 0.001.

### DETAILED DESCRIPTIONS OF THE INVENTION

The present disclosure discloses a polynucleotide, protein, biological material and use thereof in the improvement of the quality of plant fruits. Those skilled in the art can refer to the contents disclosed herein, and appropriately improve the process parameters to achieve the desired results. It should be particularly noted that, all similar substitutions and modifications are obvious to those skilled in the art, and they are deemed to be included in the present disclosure. The methods and disclosures of the present disclosure have been described through preferred embodiments. Relevant personnel can obviously modify or appropriately change and combine the methods and disclosures described herein without departing from the content, spirit and scope of the present disclosure to implement and apply the technology of the present disclosure.

The detection methods used in the following Examples are as follows.

The method for determination of SSC: more than 6 red-ripe fruits were selected from each plant, taking fresh juice from each plant, and using a portable digital display fully automatic sugar content handheld refractometer (PAL-1, ATAGO, Japan) to measure SSC at room temperature (20°C, adjusted and calibrated with distilled water before the experiment) and express it in Brix.

The method for determination of glucose, fructose, citric acid, and malic acid: six red-ripe fruits were selected from each plant, and the glucose and fructose contents were measured by using UPLC-MS/MS (ACQUITY UPLC I-Class Xevo TQ-S Micro, Waters). For detailed methods, please refer to references (R. Li, S. Sun, H. Wang, K. Wang, H. Yu, Z. Zhou, P. Xin, J. Chu, T. Zhao, H. Wang, J. Li, X. Cui, FIS1 encodes a GA2-oxidase that regulates fruit firmness in tomato. Nature Communications 11, 5844 (2020).).

Method for determination of average single fruit weight: six red-ripe fruits were selected from each plant, using an electronic balance to weigh the weight of each fruit, and calculating the average value.

This disclosure compares the genome, CDS and protein identity of CDPK8 and CDPK9 of 46 tomato varieties.

**Table 1: Identity of CDPK8 genome, CDS and protein**

| No. | Gene | Query | Chromosome | Genome identity | CDS identity | Protein identity |
|---|---|---|---|---|---|---|
| 1 | BGV006775 | CDPK8 | 11 | 100 | 93.36 | 100 |
| 2 | BGV006865 | CDPK8 | 11 | 99.71 | 95.10 | 93.357 |
| 3 | BGV007931 | CDPK8 | 11 | 99.964 | 100.00 | 95.105 |
| 4 | BGV007989 | CDPK8 | 11 | 100 | 95.63 | 100 |
| 5 | Brandywine | CDPK8 | 11 | 100 | 100.00 | 95.629 |
| 6 | EA00371 | CDPK8 | 11 | 100 | 100.00 | 100 |
| 7 | EA00990 | CDPK8 | 11 | 100 | 100.00 | 100 |
| 8 | Fla8924 | CDPK8 | 11 | 100 | 95.63 | 100 |
| 9 | Floradade | CDPK8 | 11 | 100 | 93.36 | 95.629 |
| 10 | LA2093 | CDPK8 | 11 | 99.241 | 99.53 | 93.357 |
| 11 | LYC1410 | CDPK8 | 11 | 100 | 95.57 | 99.65 |
| 12 | MM | CDPK8 | 11 | 100 | 100.00 | 95.629 |
| 13 | PAS014479 | CDPK8 | 11 | 99.187 | 100.00 | 100 |
| 14 | PI169588 | CDPK8 | 11 | 100 | 93.36 | 100 |
| 15 | PI303721 | CDPK8 | 11 | 99.982 | 93.36 | 93.357 |
| 16 | PP | CDPK8 | 11 | 99.187 | 92.89 | 93.357 |
| 17 | 1706 | CDPK8 | 11 | 100 | 100.00 | 93.007 |
| 18 | TS112 | CDPK8 | 11 | 100 | 100.00 | 100 |
| 19 | TS117 | CDPK8 | 11 | 99.638 | 92.83 | 93.007 |
| 20 | TS118 | CDPK8 | 11 | 100 | 99.53 | 99.65 |
| 21 | TS12 | CDPK8 | 11 | 100 | 97.67 | 97.552 |
| 22 | TS15 | CDPK8 | 11 | 100 | 95.16 | 95.28 |
| 23 | TS156 | CDPK8 | 11 | 100 | 100.00 | 100 |
| 24 | TS166 | CDPK8 | 11 | 100 | 95.63 | 95.629 |
| 25 | TS171 | CDPK8 | 11 | 100 | 97.73 | 97.727 |
| 26 | TS185 | CDPK8 | 11 | 100 | 100.00 | 100 |
| 27 | TS204 | CDPK8 | 11 | 100 | 100.00 | 100 |
| 28 | TS222 | CDPK8 | 11 | 100 | 97.73 | 97.727 |
| 29 | TS238 | CDPK8 | 11 | 100 | 100.00 | 100 |
| 30 | TS265 | CDPK8 | 11 | 98.734 | 97.26 | 97.378 |
| 31 | TS280 | CDPK8 | 11 | 100 | 97.73 | 97.727 |
| 32 | TS281 | CDPK8 | 11 | 100 | 100.00 | 100 |
| 33 | TS3 | CDPK8 | 11 | 100 | 97.73 | 97.727 |
| 34 | TS331 | CDPK8 | 11 | 100 | 99.53 | 99.65 |
| 35 | TS39 | CDPK8 | 11 | 99.187 | 95.57 | 95.455 |
| 36 | TS413 | CDPK8 | 11 | 99.187 | 93.30 | 93.182 |
| 37 | TS421 | CDPK8 | 11 | 98.734 | 100.00 | 100 |
| 38 | TS439 | CDPK8 | 11 | 100 | 100.00 | 100 |
| 39 | TS545 | CDPK8 | 11 | 99.187 | 99.53 | 99.65 |
| 40 | TS60 | CDPK8 | 11 | 100 | 95.63 | 95.629 |
| 41 | TS623 | CDPK8 | 11 | 99.187 | 99.53 | 99.65 |
| 42 | TS629 | CDPK8 | 11 | 100 | 97.73 | 97.727 |
| 43 | TS692 | CDPK8 | 11 | 100 | 100.00 | 100 |
| 44 | TS80 | CDPK8 | 11 | 98.734 | 100.00 | 100 |
| 45 | TS9 | CDPK8 | 11 | 100 | 97.73 | 97.727 |
| 46 | TS96 | CDPK8 | 11 | 100 | 99.53 | 99.65 |

**Table 2: Identity of CDPK9 genome, CDS and protein**

| No. | Gene | Query | Chromosome | Genome identity | CDS identity | Protein identity |
|---|---|---|---|---|---|---|
| 1 | BGV006775 | CDPK9 | 1 | 100 | 97.42 | 96.168 |
| 2 | BGV006865 | CDPK9 | 1 | 99.71 | 99.82 | 98.723 |
| 3 | BGV007931 | CDPK9 | 1 | 99.964 | 97.42 | 96.168 |
| 4 | BGV007989 | CDPK9 | 1 | 100 | 97.42 | 96.168 |
| 5 | Brandywine | CDPK9 | 1 | 100 | 97.42 | 96.168 |
| 6 | EA00371 | CDPK9 | 1 | 100 | 97.42 | 96.168 |
| 7 | EA00990 | CDPK9 | 1 | 100 | 97.42 | 96.168 |
| 8 | Fla8924 | CDPK9 | 1 | 100 | 97.42 | 96.168 |
| 9 | Floradade | CDPK9 | 1 | 100 | 97.42 | 96.168 |
| 10 | LA2093 | CDPK9 | 1 | 99.241 | 99.94 | 99.266 |
| 11 | LYC1410 | CDPK9 | 1 | 100 | 97.42 | 96.168 |
| 12 | MM | CDPK9 | 1 | 100 | 97.42 | 96.168 |
| 13 | PAS014479 | CDPK9 | 1 | 99.187 | 99.88 | 99.815 |
| 14 | PI169588 | CDPK9 | 1 | 100 | 97.42 | 96.168 |
| 15 | PI303721 | CDPK9 | 1 | 99.982 | 97.42 | 96.168 |
| 16 | PP | CDPK9 | 1 | 99.187 | 97.29 | 96.869 |
| 17 | 1706 | CDPK9 | 1 | 100 | 100.00 | 100 |
| 18 | TS112 | CDPK9 | 1 | 100 | 97.42 | 96.168 |
| 19 | TS117 | CDPK9 | 1 | 99.638 | 93.48 | 95.292 |
| 20 | TS118 | CDPK9 | 1 | 100 | 97.42 | 96.168 |
| 21 | TS12 | CDPK9 | 1 | 100 | 97.42 | 97.232 |
| 22 | TS15 | CDPK9 | 1 | 100 | 97.42 | 96.168 |
| 23 | TS156 | CDPK9 | 1 | 100 | 97.42 | 96.168 |
| 24 | TS166 | CDPK9 | 1 | 100 | 97.42 | 96.168 |
| 25 | TS171 | CDPK9 | 1 | 100 | 97.42 | 96.168 |
| 26 | TS185 | CDPK9 | 1 | 100 | 97.42 | 97.053 |
| 27 | TS204 | CDPK9 | 1 | 100 | 97.42 | 96.168 |
| 28 | TS222 | CDPK9 | 1 | 100 | 97.42 | 96.168 |
| 29 | TS238 | CDPK9 | 1 | 100 | 97.42 | 96.168 |
| 30 | TS265 | CDPK9 | 1 | 98.734 | 93.85 | 93.358 |
| 31 | TS280 | CDPK9 | 1 | 100 | 97.42 | 96.168 |
| 32 | TS281 | CDPK9 | 1 | 100 | 97.42 | 96.168 |
| 33 | TS3 | CDPK9 | 1 | 100 | 97.42 | 96.168 |
| 34 | TS331 | CDPK9 | 1 | 100 | 97.42 | 96.168 |
| 35 | TS39 | CDPK9 | 1 | 99.187 | 97.29 | 97.048 |
| 36 | TS413 | CDPK9 | 1 | 99.187 | 97.29 | 96.869 |
| 37 | TS421 | CDPK9 | 1 | 98.734 | 90.59 | 90.037 |
| 38 | TS439 | CDPK9 | 1 | 100 | 97.42 | 96.168 |
| 39 | TS545 | CDPK9 | 1 | 99.187 | 96.06 | 95.764 |
| 40 | TS60 | CDPK9 | 1 | 100 | 97.42 | 96.168 |
| 41 | TS623 | CDPK9 | 1 | 99.187 | 97.36 | 96.869 |
| 42 | TS629 | CDPK9 | 1 | 100 | 97.42 | 96.168 |
| 43 | TS692 | CDPK9 | 1 | 100 | 97.42 | 96.168 |
| 44 | TS80 | CDPK9 | 1 | 98.734 | 96.92 | 95.438 |
| 45 | TS9 | CDPK9 | 1 | 100 | 97.42 | 96.168 |
| 46 | TS96 | CDPK9 | 1 | 100 | 100.00 | 100 |

Particularly, the CDPK8 genome identity among different varieties is 98.734-100%, the CDS identity is 92.83-100%, and the protein sequence identity is 93.007-100%; the CDPK9 genome identity among different varieties is 98.734-100%, the CDS identity is 90.59-100%, and the protein sequence identity is 90.037-100%.

Note: In this disclosure, CDPK8 gene is the abbreviation of Solyc11g065660 gene, CDPK9 gene is the abbreviation of Solyc01g008440.2.1 gene, and SuSy3 gene is the abbreviation of Solyc07g042550.2.1 gene.

*CDPK8, CDPK9, SuSy3,* or CDPK8, CDPK9, SuSy3 indicating either a gene or a protein can be determined according to the context.

The reagents, instruments, strains or biological materials used in the present disclosure are all commercially available.

The present disclosure will be further described below in conjunction with Examples:

### Example 1: Knockout of CDPK8 Gene by CRISPR/Cas9 System

The protein sequence information of the tomato CDPK8 gene is as follows.

Fig. 1A shows prediction of the conserved domains of the tomato CDPK8 gene protein sequence. CDPK8 contains 533 amino acid residues. It includes a low-complexity region (LCR) at the N-terminus, a serine/threonine protein kinase domain (S_TKc) with protein kinase activity that can catalyze the phosphorylation of serine and threonine hydroxyl groups in proteins, and four EF-Hand domains at the C-terminus that can bind to calcium ions to cause changes in protein conformation. Fig. 1B shows detailed information on the location of conserved domains in the protein sequence of tomato CDPK8 protein. Data source: SMART website (http://smart.embl-heidelberg.de/).

Fig. 2A shows the genome sequence information of tomato CDPK8. The full length of the CDPK8 genome is 4914bp, including 8 exons and 7 introns. There is a 12bp sequence variation in the upstream of its promoter (324-335bp before the start codon ATG) that is closely related to the SSC. As shown in Fig. 2B, the 12bp sequence variation can divide the natural tomato population into two haplotypes. Compared with the haplotype Hap1 without the 12bp sequence variation, the SSC of the haplotype Hap2 with the 12bp sequence variation is significantly increased, indicating that the presence of the 12bp sequence can increase the SSC of tomato fruit. In addition, further analysis found that, as shown in Fig. 2C, the proportion of tomato materials with the Hap2 haplotype gradually decreased in *Solanum pimpinellifolium* (PIM), *Lycopersicon esculentum* var. *cerasiforme* A. Gray (CER) and BIG tomato (BIG) populations. In *Solanum pimpinellifolium* population, about 60% of the materials are of the Hap2 haplotype, while in the BIG tomato population, the proportion of the Hap2 haplotype is very low. Based on this, it is speculated that the deletion of the 12bp sequence may be part of the reason for the reduction in SSC during tomato domestication and improvement.

The CRISPR/Cas9 system was used to knock out the CDPK8 gene in tomatoes and obtain a CDPK8 gene knockout mutant. The specific steps are as follows.

### Step 1: Selection of sgRNA sequence

A target site sequence with a length of 19 bp was designed on the CDPK8 gene.
SEQ ID NO: 1 (CDPK8-CDS): 
ATGGGGAATTGCTGTGGGACACCTGGTAATTCTTCTGAGAATAAGAAGAAGAACAAACCAAACC
CTTTTGCTCTTGATTATGGTGCAACTCAAGCATCTGGAGGTGATGGAAACAAGCTTGTTGTGTTGAAA
GATCCAACAGGACACAATATTCAAGAAAAATATGATCTTGGTTGTGAGCTAGGAAGAGGAGAATTTG
GGGTTACATATTTATGTACTGATGTTGATACAGGGGACAAATATGCTTGCAAATCGATATCAAAGAAGA
AACTTAGGACTGCTGTAGATATAGATGATGTTAGGAGAGAAGTTGAGATCATGAAGCATTTGCCTAAA
CATCCTAATATTGTGACCTTGAAGGACACTTATGAGGATGATAATGCAGTGCATATTGTGATGGAACTT
TGTGAGGGGGGTGAGTTGTTTGATAGGATTGTTGCCAGGGGACATTATACAGAGAGAGCAGCTGCGG
TTATTATGAAGACTATAGTAGAAGTTGTTCAGATGTGTCATATGCATGGAGTAATGCATCGTGATCTCAA
ACCTGAGAACTTTCTGTTTGGTAACAAGAAAGAAACTGCTCCTTTGAAGGCTATTGACTTTGGATTAT
CAGTGTTCTTTAAACCCGGGGAACGCTTTAATGAGATTGTGGGAAGTCCTTATTACATGGCTCCAGAG
GTCCTAAAACGCAATTATGGCCCGGAGGTTGATGTCTGGAGTGCTGGAGTTATCCTTTATATTCTTCTTT
GTGGTGTTCCACCTTTCTGGGCAGAGACTGAACAAGGAGTAGCCCAAGCAATTATTCGGTCTGTGGTT
GATTTCAAGAGAGATCCATGGCCTAAGGTTTCTGATAATGCAAAGGATCTTGTAAAGAAGATGCTTGA
CCCGGATCCAACTCGACGACTCACAGCTCAGCAGGTTCTTGAGCACACCTGGTTACAAAATATAAAG
AAAGCACCAAATGTCTCATTGGGTGAGACTGTGAAAGCAAGGCTTAAACAATTTTCAGTAATGAACA
AGCTCAAGAAAAGAGCTCTAACGATTATGGCCGAGTTTTTATCAGCGGAAGAAGTGGCTGGAATGAA
GGACGCATTTGATATGATGGATACAGGAAAGAAAGGCAAGATTAACCTTGGAGAACTTAAAAATGGT
CTGCAAAAGCTTGGCCATCAGATCCCTGATGTTGATCTTCAGATTCTTATGGAAGCTGCGGATGTTGAT
GGAGATGGAAGCTTAAATTATGGGGAGTTTGTTGCTGTATCTGTTCATCTCAGAAAAATGGCAAATGAT
 
GAGCACCTGCACAAAGCATTTTCAGTTTTTGACAGAGATCAGAGTGGTTACATAGAAATCGAGGAGC
TGCGCAGTGCCTTGAGTGATGAGGATGGTGGCAACAGTGAGGAAGTCATCAATGCCATTATGCATGAT
GTTGACACTGACAAGGATGGCCGCATTAGTTACGAGGAATTTGCTGCAATGATGAAGGCTGGCACAG
ATTGGAGAAAAGCATCGAGACAGTATTCTCGTGAAAGATTCAACAGTCTCAGCTTAAAATTGATGAGA
GATGGCTCGATACAAGTCGGAAAGGAAGAAGGTAGATGA.
SEQ ID NO: 2 (CDPK8-protein): 
MGNCCGTPGNSSENKKKKNKPNPFALDYGATQASGGDGNKLVVLKDPTGHNIQEKYDLGCELGRGEFG
VTYLCTDVDTGDKYACKSISKKKLRTAVDIDDVRREVEIMKHLPKHPNIVTLKDTYEDDNAVHIVMELCE
GGELFDRIVARGHYTERAAAVIMKTIVEVVQMCHMHGVMHRDLKPENFLFGNKKETAPLKAIDFGLSVF
FKPGERFNEIVGSPYYMAPEVLKRNYGPEVDVWSAGVILYILLCGVPPFWAETEQGVAQAIIRSVVDFKR
DPWPKVSDNAKDLVKKMLDPDPTRRLTAQQVLEHTWLQNIKKAPNVSLGETVKARLKQFSVMNKLKK
RALTIMAEFLSAEEVAGMKDAFDMMDTGKKGKINLGELKNGLQKLGHQIPDVDLQILMEAADVDGDGS
LNYGEFVAVSVHLRKMANDEHLHKAFSVFDRDQSGYIEIEELRSALSDEDGGNSEEVINAIMHDVDTDKD
GRISYEEFAAMMKAGTDWRKASRQYSRERFNSLSLKLMRDGSIQVGKEEGR.
SEQ ID NO: 3 (CDPK8-genome):

ATGGGGAATTGCTGTGGGACACCTGGTAATTCTTCTGAGAATAAGAAGAAGAACAAACCAAACC
CTTTTGCTCTTGATTATGGTGCAACTCAAGCATCTGGAGGTGATGGAAACAAGCTTGTTGTGTTGAAA
GATCCAACAGGACACAATATTCAAGAAAAATATGATCTTGGTTGTGAGCTAGGAAGAGGAGAATTTG
GGGTTACATATTTATGTACTGATGTTGATACAGGGGACAAATATGCTTGCAAATCGATATCAAAGAAGA
AACTTAGGACTGCTGTAGATATAGATGATGTTAGGAGAGAAGTTGAGATCATGAAGCATTTGCCTAAA
CATCCTAATATTGTGACCTTGAAGGACACTTATGAGGATGATAATGCAGTGCATATTGTGATGGAACTT
TGTGAGGGGGGTGAGTTGTTTGATAGGATTGTTGCCAGGGGACATTATACAGAGAGAGCAGCTGCGG
TTATTATGAAGACTATAGTAGAAGTTGTTCAGGTACAGTTTTACTAATTAATGCTTGTCTTTGCTTTTCAT
CTATAATTGTTACTTGTTTGTTTATTTCATGTAATTTAGGGGTTGCTAATTTAGGATTATGTTAATGAAGT
TTGAATTGACAATGCCAACTGAAAGTGTCAATTTAGACTAGAAAGGAATGTTGAGATTGAAGTTTCAT
GTTTGTAAGCAGAATGAATGTTCTTGTCGAAGAATCTCTTTTAAGAATATCTTTCCCACTTCTTCAGTA
AATTTTCCTACTTTCTTATGTCGATAACTGCCACTCCTTTTCTGAGTTTGCCTGCACTCTCTTGGTAGAT
AGGACACTCCCTTTCTTAGCTCAGGATCTACTTTGCTTGTGTAAATGAGTGCTTAATGTTTTTATTTGAT
TCATTTTACACGTTGAGATCCTATAGCATTTAATTAGACTAGATTTTATCTTCCAATATCACTAAACCTAG
ATGTCAAATATAGTAAAAAGTTATGTTGCTCGAACTCTTCAAAGATATCGACATGTGTGTGTCAGATCC
TCCAAAAGTAGTGCATTTTTGGAGGATCCGACGGGGGTGGGGCAACAATTTTGGAGAGTTCGAGCAA
CTTAGGTAAAAAGTGTATTCATGCTCTAAATGTTGTTAGGTTTTCTATCTTTCTTATCTTGTCTTGTGAAT
TTTGCTTTCTTGGCAACTGTTTCATTGTCCTATATTTTTCTCATGCAAATACCTTTAAAATTATATACTTTC
GTGCCAACCATTTAAATGAAACTTAAGGAAGCTAATCACACAGGATTTCACCATCCAAATGTCGAAAA
TGAATCGCAATTTGTGATAGTTTTAGACAGAGAGCTTATTTTTATAGATGCAATTAGAATAATAGAAATG
TAAAATAACTATCAGTAACCAGTAAGATGCCGGTCTTTATGGTCTATTTTCAGAGGGGGAGATCTATTG
TAGCTTGCTTTCAGCATCAGTTAGTATAGCATAGGACTGTTGCTAACAGGGTTCAATTTTAAACTGTATT
TGGTGAACATAATGACATGAGAGCTCAACTTACATAACTACAGATTAGAGAGAGACATTCATTTTATGG
AATGGATTTAAGAAGTTCATGTTTCTACATTTAGTTGTTATAAGAACAGGGTGGTAACTGGTAACCGAC
GCAAACATGATGGCTAATTATCCCAAGTTTATACAATTTAATCTATGTGGTTGCTCATACATCTTTGGTC
AATGTTCACTTTACAGTTGCATTATGTTTCCTCAAGAAATGGCTGTTCTTCTTTCAATTTTCAACTTACG
GAGTTTCAATTGACATTGCAGATGTGTCATATGCATGGAGTAATGCATCGTGATCTCAAACCTGAGAAC
TTTCTGTTTGGTAACAAGAAAGAAACTGCTCCTTTGAAGGCTATTGACTTTGGATTATCAGTGTTCTTT
AAACCCGGTAATGCAATAAGTAATGATAGTGTGTTGTAACTTTTTTTCATGCTAAAAAAGATTAATGTT
GGAATCATCTTTTAATATGTTTAACAGGGGAACGCTTTAATGAGATTGTGGGAAGTCCTTATTACATGG
CTCCAGAGGTCCTAAAACGCAATTATGGCCCGGAGGTTGATGTCTGGAGTGCTGGAGTTATCCTTTATA
TTCTTCTTTGTGGTGTTCCACCTTTCTGGGCAGGTCCGTTTCTTTTATTTGCCTTCTTTAGGTAACAAGT
AATACCTCGGTTCACCGACAAATAACAAAGTAAATCAACCTTGGTGCATACTATTCATTTCTTAGAACT

TGGTAATTTATTAATGAGTGTCAAAATGAAATGGAATTTGAAACATATTGATGCGTTGTTAACATCGTC
GATGACTAGGTGAGGGTGGCTGCAAGAGTGAATCCCACCTCTTCCTGGACCAATTCAAAATCAAAGT
TCCTTTTTTTCCTTTTGGCCTGTGATAGTCAGTACTTTGTGCTTCCATTAATTTACTCACAGCTTTCTCA
GTAATTGCCTAAAATTCTACTGAATATGTGCAGAGACTGAACAAGGAGTAGCCCAAGCAATTATTCGG
TCTGTGGTTGATTTCAAGAGAGATCCATGGCCTAAGGTTTCTGATAATGCAAAGGATCTTGTAAAGAA
GATGCTTGACCCGGATCCAACTCGACGACTCACAGCTCAGCAGGTTCTTGGTAATGTTTGTTAATCCT
TGAGATGCTATACTTTCATTTGCTTGCTGTTTGTTCTCTCTTAGTTTCATAAGTTCAATTTTGTGACTTTT
TTGGGGCTTTCGTGCATATACATGAATGCAGAGCACACCTGGTTACAAAATATAAAGAAAGCACCAAA
TGTCTCATTGGGTGAGACTGTGAAAGCAAGGCTTAAACAATTTTCAGTAATGAACAAGCTCAAGAAA
AGAGCTCTAACGGTAAAATATTCCCTTTTCATTTGCAGTTACATATCATTAGCGTTCTGCACCTTCTCAG
AAGGATATCTTCATATGCCTAATTCTTAAAGCCCAAAATAAGGACAAAAGATTTTCTGCCTTCTCACAA
AAGTTTCGGGTGTTATATTCTTTCTTCATTGCTTGCACAGATTATGGCCGAGTTTTTATCAGCGGAAGA
AGTGGCTGGAATGAAGGACGCATTTGATATGATGGATACAGGAAAGAAAGGCAAGATTAACCTTGGA
GAACTTAAAAATGGTCTGCAAAAGCTTGGCCATCAGATCCCTGATGTTGATCTTCAGATTCTTATGGAA
GCTGTAAGCATTCTCATGCTCTTCATTTCACTTGATTGGATTTTCTTTCAACTTTACGAAGACTAAATAT
ATATATTTATAAGTCAAGCGTCTAATATTATTATAATTGTTCCCTTTTTTATCTTCTGCTTCACTTTTATAG
GCGGATGTTGATGGAGATGGAAGCTTAAATTATGGGGAGTTTGTTGCTGTATCTGTTCATCTCAGAAAA
ATGGCAAATGATGAGCACCTGCACAAAGCATTTTCAGTTTTTGACAGAGATCAGAGTGGTTACATAGA
AATCGAGGAGCTGCGCAGTGCCTTGAGTGATGAGGATGGTGGCAACAGTGAGGAAGTCATCAATGCC
ATTATGCATGATGTTGACACTGACAAGGTTAGAATTTTTTTTGTGTCCATTCATGTATATCTACTGGATTT
CATACTAGTGCATATACTTCTTTTATAAATGTCTCTTTAATTGCTTGTGGAACCATAAATACCCCCCTTAT
CTTGTTCCGCAGCTCCAACTACACACGAAACTATGTTGAAGACGTATTACCCCCTTGTACTTGTAAAAT
GTGTATCTTCCGTCATCCTGAGAGGCATATATGATGTAACCAAATGTGAACATTTAAGTATATGCATGTG
CTGCCTCATGTCATTTACCCCTTTTTCCCTTTTCACTTCTCTTCCATCTCCGCAACATGTTAATGTCCTCT
AGCAAATCTGAAAAAAGATGGAGTGAGCTTCTCAACGGAAAAAATCGGATTCACCCTAGTTTTAGTT
TTCTGGCAACATAAACAGCTCAAGTCGATACAAAAAAACAAATGTTCCTATCAAAAAACTCAAATTAG
TAGAAAGGAAAAGAAGGGTCATAGGTAAGGGAAGAACAAAAAGGAATAGAAAGACCCTTCGGGGT
GGCCCAGTTATTTGGGCTTGGGGCTTGGGGCTTGGGACTTCCATGTTGGAGGTCTCAAGTTCAAAACC
CCTTGCCAATGAAAGCAACGGGTTTTCCTTCTACGTTGACTGTCGAGCTCGTCGCACCGGGCTTACCT
AGTGCGGTTTACCTCTCCTTTGCGTGCTATAGGAATAGGGGTTTTACCCTGTGTGCACTCAAAGGGTA
GCGACTGCGGATTTCCCAAAAGAAAAAGGAAGAGAAAGGTAAAGAAAACATTTTAAAAAAGAGTAG
GATGGTTATAATAGAAGAAATGCTACAATATTTTTTCACCTCAGGCGCTTTAAGAGAGTAAATACTCTA
CGTTACATGGCGTCCCAATGTTGTCGTAGTTACATTTTGGACAAGAACAGGGAGTAATAGGCCTCCCG
CAAAGTTAAAATGTAGTGGGGAATGGGGGAATAATTGAGGAAGGTATTTATATATTTTCTCCTTTCTAA
ATCATATTTTCTTTTCCATTCACTGCTCTTCTGTTCAACCTTATCATTCATTTGGACTAAATGTTGAATTG
ATTTTGAGCTTTTAGTTTGTTACCACAGCTTCAAAGTTCATCAGTTACATTGTCTATAACCATGCAGGAT
GGCCGCATTAGTTACGAGGAATTTGCTGCAATGATGAAGGCTGGCACAGATTGGAGAAAAGCATCGA
GACAGTATTCTCGTGAAAGATTCAACAGTCTCAGCTTAAAATTGATGAGAGATGGCTCGATACAAGTC
GGAAAGGAAGAAGGTAGATGA.

The target site is located at positions 173-191 of SEQ ID NO: 1 (CDS sequence) and at positions 173-191 of SEQ ID NO: 3 (genomic sequence). The sequence of target site 1 is TTGGTTGTGAGCTAGGAAG (SEQ ID NO: 4 corresponding to the sequence shown in the box in SEQ ID NO: 1 and SEQ ID NO: 3).

The sgRNA sequence designed for the target site is:

GUUUUAGAGCUAGAAAUAGCAAGUUAAAAUAAGGCUAGUCCGUUAUCAACUUGAAAAAGUG
GCACCGAGUCGGUGC (SEQ ID NO: 5).

The encoding DNA molecule of this sgRNA is:

GTTTTAGAGCTAGAAATAGCAAGTTAAAATAAGGCTAGTCCGTTATCAACTTGAAAAAGTGGCAC
CGAGTCGGTGC (SEQ ID NO: 6).

### Step 2: Construction of CRISPR/Cas9 vector

The original vector contains the sgRNA sequence, and the target site 1 sequence in step 1 is further inserted into the vector to obtain a CRISPR/Cas9 vector.

### Step 3: Obtaining transgenic plants

The CRISPR/Cas9 vector obtained in step 2 was transferred to *Agrobacterium* competent cells EHA105 by heat shock transformation (*Agrobacterium* EHA105 competent cells were purchased from Shanghai Weidi Biotechnology Co., Ltd. and are available to the public) to obtain recombinant bacteria EHA105/CRISPR/Cas9.

The recombinant bacteria EHA105/CRISPR/Cas9 were then used to transform tomatoes by transformation method of *Agrobacterium-infection* (the recombinant *Agrobacterium* was propagated at 28°C, and the propagated bacterial solution was used to infect tomatoes). After kanamycin resistance screening, the T₀ generation of transgenic tomato plants were obtained.

### Step 4: Identification of transgenic plants with mutations in the CDPK8 gene

The leaves of the T₀ generation transgenic tomato plants obtained in step 3 of Example 1 were collected, and genomic DNA was extracted as a template. PCR amplification was performed by using the following primer pairs to obtain PCR amplification products of different strains.

The sequences of the primers for detection of a CDPK8 mutation sequence are as follows.

CDPK8-test-F: ATGGGGAATTGCTGTGGGAC (SEQ ID NO: 7).

CDPK8-test-R: TTCATAATAACCGCAGCTGCTCTCTCT (SEQ ID NO: 8).

The PCR amplification products of different strains were subjected to Sanger sequencing, and the sequencing results were compared with the wild-type CDPK8 gene. The CDPK8 genotypes were identified according to the following principles.

If there is a sequence with a double peak characteristic from the target site sequence, the genotype of the strain is a heterozygous genotype (the CDPK8 gene on one of the two homologous chromosomes is mutated, and the CDPK8 gene on the other chromosome is not mutated), and the strain is a T0 generation transgenic tomato heterozygous mutant strain.

If there is a double peak sequence starting from the target site sequence and the CDPK8 gene in both homologous chromosomes are mutated, then the strain is a T₀ generation transgenic tomato biallelic mutation strain.

If the sequence with a specific single-peak characteristic starting from the target site sequence is the same as the CDPK8 gene sequence of the wild-type tomato, the genotype of the strain is wild type, that is, the CDPK8 gene sequence has not mutated; if it is different from the sequence of the CDPK8 gene of the wild-type tomato, the genotype of the strain is a homozygous genotype (both the CDPK8 genes on the two homologous chromosomes are mutated), and the strain is a T₀ generation transgenic tomato homozygous mutant strain.

In this case, a homozygous mutant strain of the CDPK8 gene of the T0 generation was identified (as shown in Fig. 3), which was used to identify the tomato SSC and sugar content phenotypes described below.

Fig. 3A shows cdpk8-1 genotype 1 and cdpk8-2 genotype of the homozygous mutant strains of tomato CDPK8 gene; compared with the wild type (WT), the cdpk8-1 mutant strain has a 6-base deletion mutation, and the missing 2 amino acids are necessary for the CDPK8 protein to maintain kinase activity. After the deletion, it loses its kinase activity (as shown in Fig. 5A, mCDPK8), and the cdpk8-2 mutant strain has a 5-base deletion mutation, and the CDPK8 protein translation will undergo a frame-shift mutation, so cdpk8-1 and cdpk8-2 are both loss-of-function mutants of CDPK8 gene.

### Example 2: Knockout of CDPK9 gene by CRISPR/Cas9 system

CRISPR/Cas9 system was used to knock out the CDPK9 gene in tomatoes and obtain a CDPK9 gene knockout mutant. The specific steps are as follows.

### Step 1: Selection of sgRNA sequence

A target site sequence with a length of 19 bp was designed on the CDPK9 gene.
SEQ ID NO: 9 (CDPK9-CDS): 
ATGGGTAATTGCTGTGTGAAACCGGGTAAATCTGCCGAAAAAAAGAATAAAAAGAACAACAGCAAA
CCTAACCCTTTTTCAATTGATTATGGGGGGACTAAACATGCCTCTGGGAGTGGAAACAAGCTAGTTGT
ATTAAAAGAACCAACAGGGCAAAATATACATGATAAGTATGATTTGGGGCATGAGCTCGGAAGAGGA
GAATTTGGGGTTACTTATCTATGTACTGACTTGGAAGGAGGAGAAAAATATGCTTGCAAATCGATATCT
AAAAAGAAGCTAAGGACTGCAGTGGATATTGATGATGTTAGGAGAGAGGTTGAGATCATGAAACATT
TGCCTGTGCATCCGAATATTGTGACCTTGAAGGATACTTATGAGGATGATAATGCAGTGCACATTGTGA
TGGAATTGTGTGAGGGTGGGGAGTTGTTTGATCGAATCGTTGCTAGAGGCCACTATACAGAGAGAGC
AGCTGCTGGAATTTTGAAGACTGTCGTGGAAGTCGTTCAGATGTGTCACAGGCAAGGGGTAATGCAT
CGTGATCTCAAACCTGAGAATTTTCTTTTTGGTAACAAGAAAGAAACAGCTCCACTGAAGGCTATTGA
CTTTGGGTTGTCTGTTTTCTTTAAACCTGGGGAACGCTTTAATGAGATAGTGGGAAGTCCTTATTACAT
GGCTCCTGAGGTCCTAAAGCGCAATTATGGACCAGAGGTCGATGTCTGGAGTGCTGGAGTTATACTTT
ACATTCTTCTATGCGGTGTTCCACCTTTCTGGGCAGAGACTGAACAAGGAGTAGCCCAAGCGATTATT
CGTTCTGTGATTGATTTCAAGAGGGATCCATGGCCTAAAGTTTCTGATAATGCAAAGGATCTTGTAAAG
AAAATGCTTGATCCAGATCCAACTCGACGGCTCACAGCTCATCAAGTTCTTGAGCATCCCTGGTTACA
TAATATAAAGAAAGCACCAAACGTCTCATTGGGTGAGACTGTTAAAGCAAGACTCAAGCAGTTTTCA
GTAATGAACAAGCTCAAGAAAAAAGCTCTGACGGTTATAGCTGAGTTTTTGTCTGCGGAGGAAGTCG
CTGGAATGAAGGAAGCATTTGAAATGATGGATACCGGAAAGAAGGGCAAGATAAACCTGAATGAACT
TAAAGATGGCTTGCAGAAGCTTGGCCATCAAATCCCTGATGCTGATCTTCATATTCTCATGGAAGCGGC
TGACGTTGATGGAGATGGAAGTTTAAATTATCCAGAGTTTGTTGCTGTATCTATTCATCTTAGAAAGAT
GGCCAATGATGAACACCTGCACAAAGCATTTTCATTTTTCGACAAAAATCAGAGTGGTTTCATAGAAA
TCGAAGAGCTTCGTAGTGCTTTGAGGGATGAAGACGACAGCAACAGCGAGGAAGTCACCAATGCCAT
TATGCATGACGTTGATACAGACAAGGATGGTCGGATTAGTTATGAGGAATTTGCTGCGATGATGAAGG
CTGGTACGGACTGGAGAAAAGCATCGAGACAGTATTCTCGTGAACGTTTTAACAGTCTAAGCTTAAA
GTTGATGAGGGAAGGCTCATTACAAGTTGAAAACAAAGTCTAG.
SEQ ID NO: 10 (CDPK9-protein): 
MGNCCVKPGKSAEKKNKKNNSKPNPFSIDYGGTKHASGSGNKLVVLKEPTGQNIHDKYDLGHELGRGE
FGVTYLCTDLEGGEKYACKSISKKKLRTAVDIDDVRREVEIMKHLPVHPNIVTLKDTYEDDNAVHIVMEL
CEGGELFDRIVARGHYTERAAAGILKTVVEVVQMCHRQGVMHRDLKPENFLFGNKKETAPLKAIDFGLS
VFFKPGERFNEIVGSPYYMAPEVLKRNYGPEVDVWSAGVILYILLCGVPPFWAETEQGVAQAIIRSVIDFK
RDPWPKVSDNAKDLVKKMLDPDPTRRLTAHQVLEHPWLHNIKKAPNVSLGETVKARLKQFSVMNKLK
KKALTVIAEFLSAEEVAGMKEAFEMMDTGKKGKINLNELKDGLQKLGHQIPDADLHILMEAADVDGDG
SLNYPEFVAVSIHLRKMANDEHLHKAFSFFDKNQSGFIEIEELRSALRDEDDSNSEEVTNAIMHDVDTDKD
GRISYEEFAAMMKAGTDWRKASRQYSRERFNSLSLKLMREGSLQVENKV.
SEQ ID NO: 11 (CDPK9-genome):

ATGGGTAATTGCTGTGTGAAACCGGGTAAATCTGCCGAAAAAAAGAATAAAAAGAACAACAGCAAA
CCTAACCCTTTTTCAATTGATTATGGGGGGACTAAACATGCCTCTGGGAGTGGAAACAAGCTAGTTGT
ATTAAAAGAACCAACAGGGCAAAATATACATGATAAGTATGATTTGGGGCATGAGCTCGGAAGAGGA

GAATTTGGGGTTACTTATCTATGTACTGACTTGGAAGGAGGAGAAAAATATGCTTGCAAATCGATATCT
AAAAAGAAGCTAAGGACTGCAGTGGATATTGATGATGTTAGGAGAGAGGTTGAGATCATGAAACATT
TGCCTGTGCATCCGAATATTGTGACCTTGAAGGATACTTATGAGGATGATAATGCAGTGCACATTGTGA
TGGAATTGTGTGAGGGTGGGGAGTTGTTTGATCGAATCGTTGCTAGAGGCCACTATACAGAGAGAGC
AGCTGCTGGAATTTTGAAGACTGTCGTGGAAGTCGTTCAGGTATAGTTTTTATCAACTTCTTAGTTCTC
AGTTACCTACTGCTTTTATTACCTTTTGCTTAGGTTATCTTATTATATCCATTATTTTCAGCATAGCTTCTT
CATTACTGTATTTCCTTTTCATATTGTTTTTATATATGTCTTACTTAGGCTGAGGGTCCATTGGAAATAGC
CTCTCTACCTTCACAAGGTAGGAGTAAGGCTGCGTACACACTACCCTTCCCACACCCCACTTGTGGGA
CTATACTAAGTATGTTGTTGTTTTAGTTCTCAATTGATGCTTTTTGGTTTTTATGTAGTAACTTGTGGTGT
TTCTTTTACTGAGTTCTTCAAAGCTTTTGTATGGGTTTTATATCATGACGGATGTATCCTGTATTGTTGTT
TCGGGTTTTTATTAAAGAAGTCTGGCAATTTCAAGTTAAAAAGAAAATCATATCTTAAAAGAGAAGCC
TAATCTTGAGCAAATCTTGAGCAGATAGCAATTTCAGGGTGAAATGTGAATATTCTTGGTGAAGTTGC
ATATGAACTGGTAACCATTATTCAATTCATTTCTAGTGTTGATCTTTGCAATCAATGGTTTCATTCTAGTG
TACGTTCATGCTGTAGCATTTACCTTGATATCCTCATGGAATTTCCTTTACTGCCAAAATTATAATGAGA
AGTGGTTATCTTTTTTAATAATGGTAAAGTATGAGAAGAGGTTTTCTTATGTTCTCAGTCTTTGCTAGTT
CTATTTCTTTACTCGTTTTACTTAACTTTTTTAGTTTCTTGTGAACTGTAGTATTGCTCTATATCTTCTCAC
TCAAATTCATGTTCTAAATCTTAACCATTCTAGTGATATAAATGAAGTTAATTGGTGATATAGGATATCCC
CCTCCAAAATCTAACAATCTTTCAACTCATCATAGTCTTGGGCAGATAGCTAGTTAGAACCGTAACAAA
TATTTAGGAAAGTAGATCATTCGAAATAGTTATCCTTGTGAAATATTATTCTTTATCTTCATTATTGACCA
TTGGTTTTCAAACTCTAGTACTCTTAAGTGGAAGACTCACTTCTTGTTTCTGTGGAGTATTAGCTATAG
CGGCCTACAACAGAATTTGATTGTTGCACTCAAAGTTAAACAGAGTTCTACAGTTTAGGGAAATGATA
ATGGAATTTAAATTTAGTTTGTATACTGAAGTAAATATGGTTGACACTATCAGAAACAAGTGCTTTTTAC
TTCAAATATTGATCTTTATCCTCATTGCGAACCATTGATGTCCAAACTGTTTTAGATGGAAGAGCAATTT
GTTGTTCATTTGAGTGGAATAGTCACCAACAACAGCTAATTAACTACGTATAGCTTAGAGAGATGCGA
ACCAATGGTAAATTTAAGTTGTCTCCTGAAGTAGTTACTGATTGTTCATGATAAAGGTCATTAAAGTTG
AATTCTCTATTTAGTCAGTGTCCTTTTCTGCATCGACTATATAGTGGCCTGAATGACATTACAAAATATTT
ATGAGTTGGTAGGAATCTTTTTTTCATATGTGAATCATGAATGTGTTCACAAATGATCAATCAGAGAGT
GATTATTGTATTATGTGGTGTTGATAACTTTTGAACTTTTCTTCATTTGGTTTGTGTTTAACTGTTATCTG
AAAGGCTTGGTTGGTGGTACTAAAGCTATTGGATTAGCAGAAAACTTTATACATGTTTATGGTCATGTT
TCGCTCTCCAGTCCCCGTTTTTATTTGTCAAAGGTCAGTTCATATATTTCCAATGGTTTTGCAGATGTGT
CACAGGCAAGGGGTAATGCATCGTGATCTCAAACCTGAGAATTTTCTTTTTGGTAACAAGAAAGAAA
CAGCTCCACTGAAGGCTATTGACTTTGGGTTGTCTGTTTTCTTTAAACCTGGTAATGGAATTGGAAAA
AAAACAGTGAGTTTAAGACTTTTATCATGCTAAACAAAGGTTTAACATGTTGATTATTTTAACAGGGGA
ACGCTTTAATGAGATAGTGGGAAGTCCTTATTACATGGCTCCTGAGGTCCTAAAGCGCAATTATGGACC
AGAGGTCGATGTCTGGAGTGCTGGAGTTATACTTTACATTCTTCTATGCGGTGTTCCACCTTTCTGGGC
AGGTCTGCTTATTTTATTTCTTCGTTTTTTATAACCATGGAACGAACCTCAATGCAGATATTTTCATGTG
CAATTTTTGGAACTTGATAATTGATTAACTAAGATTGTGAATATGAAGTAGAACTTGATTCATGTTAGA
ATATGTTGTTGAAATATTAGATGATGAGGCAAGTGAGGTGAAAGACTGTCCCTGCTTTAATCAATGTAG
ATCATGTTCAATTATTTTGGTTTCTCTTAATGGTTCTTATGGGTTGGACTTTGCTAAATAACTTGCATAAA
TATGTTAACATATTCTTGATACTTCCCTTAAGTTCCTCTGAATATTTGCAGAGACTGAACAAGGAGTAG
CCCAAGCGATTATTCGTTCTGTGATTGATTTCAAGAGGGATCCATGGCCTAAAGTTTCTGATAATGCAA
AGGATCTTGTAAAGAAAATGCTTGATCCAGATCCAACTCGACGGCTCACAGCTCATCAAGTTCTTGGT
AATGTTTGTGGAATATTGAGATATTGTACTTTATTCATTCGTTGTTTTACTCTCTCTCTCTCTCTCTCTCT
CTCTCTATCTATCTCTGATAAGTATTTTTTTCCGGGGCTTTGACTCTTGTGGTGCAGAGCATCCCTGGTT
ACATAATATAAAGAAAGCACCAAACGTCTCATTGGGTGAGACTGTTAAAGCAAGACTCAAGCAGTTT
TCAGTAATGAACAAGCTCAAGAAAAAAGCTCTGACGGTAAGCACACACTTAGTCTGCAATCATATTTA
TAAGCTCTGGATTCTTCACTGTCCACTAAAGATGAATTCATATGCTTTTCCTCATAGATCGAAATGAAG
GAGAGAAGGTTGTGTCTTAATTATTTACTGCACTGCCTTGTGCTATTTAATAAAAACTTAATTCTCATCG

TAAAAAATGAGAGGCGGTTCTCAAAATTTTGATCAAGTTTCGTTTTTTTATCTTGATATATGTACTTTCT
TGTATTCCAAACAGGTTATAGCTGAGTTTTTGTCTGCGGAGGAAGTCGCTGGAATGAAGGAAGCATTT
GAAATGATGGATACCGGAAAGAAGGGCAAGATAAACCTGAATGAACTTAAAGATGGCTTGCAGAAGC
TTGGCCATCAAATCCCTGATGCTGATCTTCATATTCTCATGGAAGCGGTAAGCGGTCTCATGCCCTGCT
TCTTGGCTCGATCAGATTTCTTATAGCTCTACCGGGGGTAACATAATATTTGAAAGTTAGCTGCTAAACT
TTTCTGCCTCAAAGAGAAAACAGGTGCCTGAGATGATATGGCCATGTCTTACGTAGAACTGTAAATTA
CATCAATGGACATTATTTTAAAATGACCGAAAACTACATGGAATCCATGTGAACTTAGTAAAGAAAATA
TTCAATGGAAGTAAAGGATACCAAGTAGTTGGATTAAGGCTAGATTGTTGGTGGTACACTGACATTAG
TGTCTCAATGTTTACTAAGAGTCTCAGTTTGATTAGAGATTTGCACGTAGATGCAGGGATAACCGTGAG
ATTAAGAAAATTTTAAGAAATTCACGGAGATGAAAAAGATCTGTAATACTCATATAGCTTAGGGCCAA
GCCCAACTAGTTTGGGATCAAATCGTAGTTGTTGTTGATAAGAATTTAGCATTAGACTTTTGTCTGTCA
CCCTACTGTCAAAAAATTGATTGATTACAAAATATATTTTGTCAATATAAGTTGTAACATTTTTCTACCAT
TTCAATGTAGAAAAAAGGAAAATCTGTAGCAAAATTAGCTTGACATTTCGCTTGTCTTAGCATCCCTG
GTTCAATGCTTATTATTATCCAGAAAGGAAAAAAAAACATTTCTCTTGATCCTTTTGCATCTTCGACC
TGAAGGATGCTTATCTTTTTTATTATTTTATTTCCTTGAACTTTTGTTTTATGTTTTTCTTATTCTGTATCG
GTTTTACAGGCTGACGTTGATGGAGATGGAAGTTTAAATTATCCAGAGTTTGTTGCTGTATCTATTCATC
TTAGAAAGATGGCCAATGATGAACACCTGCACAAAGCATTTTCATTTTTCGACAAAAATCAGAGTGGT
TTCATAGAAATCGAAGAGCTTCGTAGTGCTTTGAGGGATGAAGACGACAGCAACAGCGAGGAAGTCA
CCAATGCCATTATGCATGACGTTGATACAGACAAGGTCAGTATGTCCATTTATGTGCACGTACACCATT
TAGTAACTTTTCGTTCTTTGGTTCATACTTCATAGTGCAAATATTATGCATTGTTGCTAATGTTTACATCT
TAATGAGTTCTCATTTTGAAGTTCTCTTCTGAACCCTCGTTTGAGTTTAGAGCTCAAATACTCCACCTA
GTTGTGACTTATTGTATCTCTAAAGTCCGTCATATTATATAAACGTATTATACTTTGATGGCATGCACACT
TTCTAGCACATATGTGTTGCTAACGAAACTCTAGTGCTGTGACAAATGAAATACATGTATACATTGATG
GCATCTGTTCTTCCCATCACATATGTACTACCGGTCATTACTCTCTAGTGCTGTGACATTATGCTCTCCC
TGTTCGCTTCATGAGTTACACGGTTGGTTAACCTGCTGCTCGTTACATGTCTGCTACAGAATTCAAGTT
TTGTTAGCTGTTTTCCATCTCATCTCTAACAACATCCCCACTCATTTGTATTAGCTTCTTATCTGGAGTTT
TGTGAGCTCAGTCTTGTATCGTCTTGTGTCAAAACGTGTGCAGGATGGTCGGATTAGTTATGAGGAATT
TGCTGCGATGATGAAGGCTGGTACGGACTGGAGAAAAGCATCGAGACAGTATTCTCGTGAACGTTTT
AACAGTCTAAGCTTAAAGTTGATGAGGGAAGGCTCATTACAAGTTGAAAACAAAGTCTAG.

The target site is located at positions 179-197 of SEQ ID NO: 9 (CDS sequence) and at positions 179-197 of SEQ ID NO: 11 (genomic sequence), and the target site 2 sequence is TGGGGCATGAGCTCGGAAG (SEQ ID NO: 12, for example, the in-frame sequence in SEQ ID NO: 9 and SEQ ID NO: 11).

The sgRNA sequence designed for the target site is:

GUUUUAGAGCUAGAAAUAGCAAGUUAAAAUAAGGCUAGUCCGUUAUCAACUUGAAAAAGUG
GCACCGAGUCGGUGC (SEQ ID NO: 13).

The encoding DNA molecule of this sgRNA is:

GTTTTAGAGCTAGAAATAGCAAGTTAAAATAAGGCTAGTCCGTTATCAACTTGAAAAAGTGGCAC
CGAGTCGGTGC (SEQ ID NO: 14).

### Step 2: Construction of CRISPR/Cas9 vector

The original vector contains the sgRNA sequence, and the target site 2 sequence in step 1 was further inserted into the vector to obtain a CRISPR/Cas9 vector.

### Step 3: Obtaining transgenic plants

The CRISPR/Cas9 vector obtained in step 2 was transferred to *Agrobacterium* competent cells EHA105 by heat shock transformation (*Agrobacterium* EHA105 competent cells were purchased from Shanghai Weidi Biotechnology Co., Ltd., and are available to the public) to obtain recombinant bacteria EHA105/CRISPR/Cas9.

The recombinant bacteria EHA105/CRISPR/Cas9 were then used to transform tomatoes by transformation method of *Agrobacterium-infection* (the recombinant *Agrobacterium* was propagated at 28°C, and the propagated bacterial solution was used to infect tomatoes). After kanamycin resistance screening, the T₀ generation transgenic tomato plants were obtained.

### Step 4: Identification of transgenic plants with mutations in the CDPK9 gene

The leaves of the T₀ generation transgenic tomato plants obtained in step 3 of Example 2 were collected, and genomic DNA was extracted as a template. PCR amplification was performed by using the following primer pairs to obtain PCR amplification products of different strains.

The sequences of primers for detection of the CDPK9 mutation sequence are as follows.

CDPK9-test-F: AGAACAACAGCAAACCTAACCC (SEQ ID NO: 15).

CDPK9-test-R: TCCAGCAGCTGCTCTCTCTG (SEQ ID NO: 16).

The PCR amplification products of different strains were subjected to Sanger sequencing, and the sequencing results were compared with the wild-type CDPK9 gene. The CDPK9 genotypes were identified according to the following principles.

If there is a sequence with a double peak characteristic from the target site sequence, the genotype of the strain is a heterozygous genotype (the CDPK9 gene on one of the two homologous chromosomes is mutated, and the CDPK9 gene on the other chromosome is not mutated), and the strain is a T₀ generation transgenic tomato heterozygous mutant strain.

If there is a double peak sequence starting from the target site sequence and the CDPK9 gene in both homologous chromosomes are mutated, then the strain is a T₀ generation transgenic tomato biallelic mutation strain.

If the sequence with a specific single-peak characteristic starting from the target site sequence is the same as the CDPK9 gene sequence of the wild-type tomato, the genotype of the strain is wild type, that is, the CDPK9 gene sequence has not mutated; if it is different from the sequence of the CDPK9 gene of the wild-type tomato, the genotype of the strain is a homozygous genotype (both the CDPK9 genes on the two homologous chromosomes are mutated), and the strain is a T₀ generation transgenic tomato homozygous mutant strain.

In this case, a homozygous mutant strain of the CDPK9 gene of the T₀ generation was identified (as shown in Fig. 3), which was used to identify the tomato SSC and sugar content phenotypes described below.

Fig. 3B shows the cdpk9-1genotype of the homozygous mutant strain of the tomato CDPK9 gene; compared with the wild type (WT), the mutant strain has a 5-base deletion mutation, and the CDPK9 protein translation will undergo a frame-shift mutation, so cdpk9-1 is a loss-of-function mutant of CDPK9 gene.

### Example 3: Determination of SSC, Sugar and Acid Content in Fruits of CDPK8 and CDPK9 Gene Knockout Tomato Mutants

The T₂ generation CDPK8 homozygous mutant strains cdpk8-1 and cdpk8-2, and the CDPK8 and CDPK9 homozygous double gene mutant strains cdpk8-2/cdpk9-1 obtained in Examples 1 and 2 were planted in the greenhouse of the Shouguang Experimental Base of the Institute of Vegetables and Flowers, Chinese Academy of Agricultural Sciences in the autumn of 2021. The detection results of SSC, sugar and acid content and average single fruit weight of wild-type plants and mutants are shown in Fig. 4.

Through systematic identification of the SSC, sugar and acid contents of the mutants, it was found that: the fruit SSC, glucose, fructose and citric acid contents of the homozygous mutant of CDPK8 and the homozygous double gene mutant strains of CDPK8 and CDPK9 are increased, the malic acid content is decreased, and the single fruit weight does not change significantly, indicating that the CDPK8 and CDPK9 genes play a key role in regulating the contents of SSC, glucose, fructose, citric acid and malic acid in tomato fruits.

### Example 4: CDPK Protein can Phosphorylate SuSy3 Protein and Promote Its Degradation

In vitro phosphorylation experiments and protein degradation experiments demonstrated that, CDPK protein can phosphorylate tomato sucrose synthase SuSy3 and promote its degradation. The specific steps are as follows.

### Step 1. In vitro expression of SuSy3 and CDPK8 proteins

The SuSy3-HIS and CDPK8-HIS expression vectors were constructed, and the SuSy3-HIS and CDPK8-HIS proteins were induced to express in vitro and were purified.
SEQ ID NO: 17 (SuSy3-CDS): 
ATGGCTCAACGTGTTCTAACTCGTGTTCACAGTCTTCGTGAACGTCTTGATGCTACTTTGGATGCTCAT
CGCAATGAAATTTTGCTCTTTCTTTCAAGGATCGAAAGCCACGGGAAAGGGATCTTGAAACCTCACCA
GCTACTGGCTGAGTTTGAATCAATTCAGAAAGAAGACAAAGACAAACTGAATGATCATGCCTTTGAA
GAAGTCCTGAAATCCACTCAGGAAGCAATTGTTTTGCCCCCATGGGTTGCACTTGCTATTCGTTTGAG
GCCCGGTGTGTGGGAATATGTCCGTGTGAATGTTAATGCTCTTAGTGTTGAGGAGCTGACTGTGCCTG
AGTTTTTGCAATTCAAGGAAGAACTTGTTAACGGAACTTCCAGTGATAACTTTGTTCTTGAATTGGATT
TTGAGCCCTTCACTGCATCATTTCCAAAACCAACCCTCACGAAATCAATTGGAAATGGAGTTGAATTC
CTCAACAGGCACCTCTCTGCTAAAATGTTCCATGACAAGGAAAGCATGACCCCTCTTCTCGAGTTTCT
TCGAGTTCACCACTACAATGGAAAGTCAATGATGCTGAATGATAGAATTCAGAATTTGTATACTCTCCA
AAAAGTCCTGAGGAAGGCCGAGGAATACCTCACCACCCTTTCGCCAGAAACTTCATACTCCTCATTTG
AGCACAAGTTCCAAGAAATTGGCTTGGAGAGAGGTTGGGGTGACACCGCAGAGCGTGTTCTAGAGA
TGATCTGCATGCTCCTGGATCTCCTTGAGGCTCCTGACTCATGTACTCTTGAGAAGTTCCTTAGTAGAA
TTCCTATGGTTTTCAATGTAGTTATACTTTCACCTCATGGATATTTCGCCCAGGAAAATGTCTTGGGTTA
CCCCGACACTGGTGGTCAGGTTGTCTATATTTTGGATCAAGTTCCTGCCTTGGAGCGTGAGATGCTCA
AGCGCATAAAGGAGCAAGGACTTGATATCAAACCGCGTATTCTTATTGTTACTCGGCTTCTCCCTGATG
CAGTTGGTACCACTTGTGGTCAGCGACTCGAGAAGGTATTTGGAACTGAGCATTCACATATTCTTAGG
GTCCCCTTTAGGACTGAAAAGGGCATTGTTCGCAAATGGATCTCTCGTTTTGAAGTCTGGCCATACAT
GGAGACTTTCATTGAGGATGTGGGGAAAGAAATAACCGCAGAACTGCAAGCTAAGCCAGATCTTATT
ATTGGAAACTATAGTGAGGGAAACCTTGCAGCCTCCTTGTTGGCTCACAAGTTAGGTGTAACACAGTG
CACCATTGCTCATGCATTGGAGAAAACCAAATATCCTGATTCTGACATTTACTTGAACAAATTTGACGA
GAAATACCACTTCTCAGCTCAGTTCACAGCTGATCTTATAGCAATGAATCATACTGATTTCATTATCACC
AGCACCTTCCAGGAGATAGCAGGAAGCAAGGACACTGTTGGACAGTATGAGAGCCACATGGCCTTCA
CAATGCCTGGATTGTATAGAGTTGTTCATGGCATTGATGTGTTCGACCCCAAATTCAACATTGTGTCAC
CAGGAGCTGATGTGAATCTCTATTTCCCATACTCCGAAAAGGAAAAGAGATTGACAACTTTTCACCCT
GAAATTGAAGACTTGCTGTTTAGCGATGTTGAGAACGAAGAACACCTGTGTGTGTTGAAGGACAGGA
ATAAGCCCATCATATTCACCATGGCAAGATTGGACCGAGTGAAGAACTTAACTGGACTTGTCGAGTGG
TATGCTAAGAATCCACGACTAAGGGAGTTGGTTAACCTTGTAGTGGTTGGTGGAGACCGAAGAAAGG
AATCCAAAGACTTGGAAGAGCAGGCAGAGATGAAGAAGATGTATGAACTTATAAAGACTCACAATTT
GAATGGCCAGTTCCGATGGATTTCTTCCCAGATGAACCGCGTGAGGAATGGGGAACTCTACAGGTAC
ATTGCTGACACAAGGGGAGCTTTCGTGCAGCCTGCATTCTACGAGGCTTTCGGTCTGACTGTTGTTGA
GGCCATGAGCTGCGGTTTGCCTACATTTGCAACTAATCAAGGTGGTCCAGCTGAGATCATCGTTCATG
GAAAGTCTGGTTTCCAAATTGATCCATACCATGGCGAGCAGGCTGCTGATCTCCTCGCTGAGTTCTTC
GAGAAATGTAAGGTAGACCCTTCACATTGGGAAGCCATTTCCAAGGGTGGCCTTAAGCGTATACAGGA
GAAGTACACATGGCAAATCTACTCCGACCGGCTGTTGACACTAGCTGCTGTTTACGGGTTCTGGAAGC
ACGTTTCCAAGCTTGATCGTCTTGAAATTCGTCGTTATCTTGAGATGTTTTACGCTCTCAAATTCCGCA
 AGCTGGCTGAACTTGTCCCATTGGCTGTTGAGTAA.
SEQ ID NO: 18 (SuSy3-protein): 
MAQRVLTRVHSLRERLDATLDAHRNEILLFLSRIESHGKGILKPHQLLAEFESIQKEDKDKLNDHAFEEVL
KSTQEAIVLPPWVALAIRLRPGVWEYVRVNVNALSVEELTVPEFLQFKEELVNGTSSDNFVLELDFEPFTA
SFPKPTLTKSIGNGVEFLNRHLSAKMFHDKESMTPLLEFLRVHHYNGKSMMLNDRIQNLYTLQKVLRKA
EEYLTTLSPETSYSSFEHKFQEIGLERGWGDTAERVLEMICMLLDLLEAPDSCTLEKFLSRIPMVFNVVILS
PHGYFAQENVLGYPDTGGQVVYILDQVPALEREMLKRIKEQGLDIKPRILIVTRLLPDAVGTTCGQRLEKV
FGTEHSHILRVPFRTEKGIVRKWISRFEVWPYMETFIEDVGKEITAELQAKPDLIIGNYSEGNLAASLLAHK
LGVTQCTIAHALEKTKYPDSDIYLNKFDEKYHFSAQFTADLIAMNHTDFIITSTFQEIAGSKDTVGQYESH
MAFTMPGLYRVVHGIDVFDPKFNIVSPGADVNLYFPYSEKEKRLTTFHPEIEDLLFSDVENEEHLCVLKDR
NKPIIFTMARLDRVKNLTGLVEWYAKNPRLRELVNLVVVGGDRRKESKDLEEQAEMKKMYELIKTHNLN
GQFRWISSQMNRVRNGELYRYIADTRGAFVQPAFYEAFGLTVVEAMSCGLPTFATNQGGPAEIIVHGKSGF
QIDPYHGEQAADLLAEFFEKCKVDPSHWEAISKGGLKRIQEKYTWQIYSDRLLTLAAVYGFWKHVSKLD
RLEIRRYLEMFYALKFRKLAELVPLAVE.
SEQ ID NO: 19 (SuSy3-genome):

ATGGCTCAACGTGTTCTAACTCGTGTTCACAGTCTTCGTGAACGTCTTGATGCTACTTTGGATGCTCAT
CGCAATGAAATTTTGCTCTTTCTTTCAAGGTATGGTCTTACAGCCATGTTTTCGCTTTTTGTAATTTTCG
TTTGGTATGTTTCGCAAGCCTAAATAGTTTTGGGGATACAAACTTAGTACTGCCTTATTCCTTTGTTTGG
TTATTGATCTCGGAATAAGTTATATCAGAATTAGTAATTAGTACCTCGATAAGTTATCTCTGATGGAATAA
TAGTACTGTTAACTAGTAATCCTGGAACAATACAATGAAAATGACAAAAATCCCCCAGAGGTCCCTTA
GATCCTTTTTTACTTAATAAGGTGGAAGGTATTCTCGTAAACAAATAACTTTCGTTCTTAAAAATTATGC
AATGAATGTTTGTCCTAGTAAAACTTGTCTTCAAACCAAACGACCCCTAATTGACGGACAAATATATGG
TTCCATCAAGTACATCATGAGTTATTCTTGCTGTTTTTGGCTGTATCAGGATCGAAAGCCACGGGAAAG
GGATCTTGAAACCTCACCAGCTACTGGCTGAGTTTGAATCAATTCAGAAAGAAGACAAAGACAAACT
GAATGATCATGCCTTTGAAGAAGTCCTGAAATCCACTCAGGTAACTTGGTTTTGTTGTTAGGTTGATTA
TATTTAATTTTCTTACTAAGCTAACATGTGTATCTGTTTGTACTTGCATTTGAAATTTTCTTTTGCGTTGT
TCGTCATACTAGGAAGCAATTGTTTTGCCCCCATGGGTTGCACTTGCTATTCGTTTGAGGCCCGGTGTG
TGGGAATATGTCCGTGTGAATGTTAATGCTCTTAGTGTTGAGGAGCTGACTGTGCCTGAGTTTTTGCAA
TTCAAGGAAGAACTTGTTAACGGAACGTAAGTTTTACGTTTGAATTTGATGATGAGTTATCTAATCAAC
ATGTTCTTAGAATCTTTTTGATTAATGTTGTGATTTTCCCTGATGCAGTTCCAGTGATAACTTTGTTCTT
GAATTGGATTTTGAGCCCTTCACTGCATCATTTCCAAAACCAACCCTCACGAAATCAATTGGAAATGG
AGTTGAATTCCTCAACAGGCACCTCTCTGCTAAAATGTTCCATGACAAGGAAAGCATGACCCCTCTTC
TCGAGTTTCTTCGAGTTCACCACTACAATGGAAAGGTAAATTTAGTGTTATGGTGGTTTTTCTCCGATA
AATTTCGAGTATGGAGTTACTGATTTTTGCATCCAACAAATGATTAACATGTTCGAACAATCATTTTCTG
TGCAGTCAATGATGCTGAATGATAGAATTCAGAATTTGTATACTCTCCAAAAAGTCCTGAGGAAGGCC
GAGGAATACCTCACCACCCTTTCGCCAGAAACTTCATACTCCTCATTTGAGCACAAGTTCCAAGAAAT
TGGCTTGGAGAGAGGTTGGGGTGACACCGCAGAGCGTGTTCTAGAGATGATCTGCATGCTCCTGGAT
CTCCTTGAGGCTCCTGACTCATGTACTCTTGAGAAGTTCCTTAGTAGAATTCCTATGGTTTTCAATGTA
GTTATACTTTCACCTCATGGATATTTCGCCCAGGAAAATGTCTTGGGTTACCCCGACACTGGTGGTCAG
GTGCATTGCTTATTTGTGATCACTTTGTCTTACTCCTTAGAAACCTATTGGGGCAAGTGCTGAGTTCCC
GTTCCTTTAATTTGCAGGTTGTCTATATTTTGGATCAAGTTCCTGCCTTGGAGCGTGAGATGCTCAAGC
GCATAAAGGAGCAAGGACTTGATATCAAACCGCGTATTCTTATTGTTAGTATTCTCAGAGATTGTGCTT
ATAGATTATGATTATGCAGGATTTTGATTTGTTCTAATGCAACATCTGATCTTTGTTTAAATTCTCAGGTT
ACTCGGCTTCTCCCTGATGCAGTTGGTACCACTTGTGGTCAGCGACTCGAGAAGGTATTTGGAACTGA
GCATTCACATATTCTTAGGGTCCCCTTTAGGACTGAAAAGGGCATTGTTCGCAAATGGATCTCTCGTTT
TGAAGTCTGGCCATACATGGAGACTTTCATTGAGGTGAAGCAACTTTTCTGTATTCATTTTTCAGTCTT
CTAGTTGATTTTTGCAGCAATTTTCTACTTACACTAAAACTGTGACTTTAATACATTAGGATGTGGGGA
AAGAAATAACCGCAGAACTGCAAGCTAAGCCAGATCTTATTATTGGAAACTATAGTGAGGGAAACCTT

GCAGCCTCCTTGTTGGCTCACAAGTTAGGTGTAACACAGGTCTGTAACATTGGTCACATGTATAAGATT
GACTTTGCATTTCCTTTCATTTGGAACTCGGAGTTATTAAGAATTCTCTTTTGCGTTGATCTGCAGTGC
ACCATTGCTCATGCATTGGAGAAAACCAAATATCCTGATTCTGACATTTACTTGAACAAATTTGACGAG
AAATACCACTTCTCAGCTCAGTTCACAGCTGATCTTATAGCAATGAATCATACTGATTTCATTATCACCA
GCACCTTCCAGGAGATAGCAGGAAGGTATGACATCAATTCGCTCTATGATTAAATTGTTCTTCTTCCTG
TTTCATTGTGTTTGATCCTAAAACATTTTTCAATTTTCTTTAGCAAGGACACTGTTGGACAGTATGAGA
GCCACATGGCCTTCACAATGCCTGGATTGTATAGAGTTGTTCATGGCATTGATGTGTTCGACCCCAAAT
TCAACATTGTGTCACCAGGAGCTGATGTGAATCTCTATTTCCCATACTCCGAAAAGGAAAAGAGATTG
ACAACTTTTCACCCTGAAATTGAAGACTTGCTGTTTAGCGATGTTGAGAACGAAGAACACCTGTATGT
TTCTATAATAGTGCAACATTGTGTTTACTGATATAAACAAGCTTGTACTGAAATAGATTTGTGTTCCTAT
ATCAGGTGTGTGTTGAAGGACAGGAATAAGCCCATCATATTCACCATGGCAAGATTGGACCGAGTGAA
GAACTTAACTGGACTTGTCGAGTGGTATGCTAAGAATCCACGACTAAGGGAGTTGGTTAACCTTGTAG
TGGTTGGTGGAGACCGAAGAAAGGAATCCAAAGACTTGGAAGAGCAGGCAGAGATGAAGAAGATGT
ATGAACTTATAAAGACTCACAATTTGAATGGCCAGTTCCGATGGATTTCTTCCCAGATGAACCGCGTG
AGGAATGGGGAACTCTACAGGTACATTGCTGACACAAGGGGAGCTTTCGTGCAGCCTGCATTCTACG
AGGCTTTCGGTCTGACTGTTGTTGAGGCCATGAGCTGCGGTTTGCCTACATTTGCAACTAATCAAGGT
GGTCCAGCTGAGATCATCGTTCATGGAAAGTCTGGTTTCCAAATTGATCCATACCATGGCGAGCAGGC
TGCTGATCTCCTCGCTGAGTTCTTCGAGAAATGTAAGGTAGACCCTTCACATTGGGAAGCCATTTCCA
AGGGTGGCCTTAAGCGTATACAGGAGAAGTAAGCTGCTCATCTTTTCGTCCTATCACGTGATCACTATT
GAGTGCATTAATTCAAAGTGATTCTAATCTCCTGTTGCTGTAGGTACACATGGCAAATCTACTCCGACC
GGCTGTTGACACTAGCTGCTGTTTACGGGTTCTGGAAGCACGTTTCCAAGCTTGATCGTCTTGAAATT
CGTCGTTATCTTGAGATGTTTTACGCTCTCAAATTCCGCAAGCTGGTATGTCTCTCTGCTTTCTGCACTT
GTCCAATAGTCTAAAGCAACTGTTAATGGAGCTTCTGTATTTTGTTTTGATTCAGGCTGAACTTGTCCC
ATTGGCTGTTGAGTAA.

### Step 2: In vitro phosphorylation experiments prove that CDPK8 can phosphorylate SuSy3 protein

The in vitro expressed CDPK8-HIS protein or mCDPK8-His protein without kinase activity was incubated with SuSy3-HIS protein in kinase reaction buffer (50 mM Tris-HCl, pH 7.5, 10 mM MgCl₂, 1 mM DTT, 5 µCi γ-³²P-ATP) at room temperature for 30 minutes. Subsequently, 5× SDS loading buffer (250 mM Tris-HCl, pH 6.8, 10% [w/v] SDS, 0.5% bromophenyl blue, 50 mM DTT) was added to terminate the reaction, and the samples were separated by 10% SDS-PAGE gel. After electrophoresis, Coomassie Brilliant Blue R250 was used for staining as a protein loading control, and phosphorylated proteins were observed by autoradiography. In vitro phosphorylation experiments demonstrated that: the CDPK8 protein itself has kinase activity and can phosphorylate the SuSy3 protein, while the mCDPK8 protein lacking two key amino acids has no kinase activity and cannot phosphorylate the SuSy3 protein (Fig. 5A). The products of co-incubation of SuSy3-HIS and CDPK8-HIS proteins, and the products of incubation of SuSy3-HIS protein alone were used as negative controls. Mass spectrometry analysis showed that: 14 amino acid residues on the SuSy3 protein can be phosphorylated by CDPK8 protein, and the sites are T7, S11, T19, S152, S165, S174, T176, S191, S219, S360, T430, S535, T543, and S706 in sequence.

### Step 3: In vitro protein degradation experiments proved that phosphorylation of SuSy3 by CDPK promotes its degradation

The total protein of wild-type tomato and cdpk8-1 mutant fruits at the red-ripe stage was extracted by using degradation buffer (300 mM Tris-HCl pH 8.0, 600 mM NaCl, 4 mM MgCl₂, 20 mM DTT) respectively, and then equal amounts of SuSy3-His recombinant protein and 5 mM ATP were added to incubate at room temperature (25°C) for 0 h, 1 h, 2 h and 4 h. Then 5×SDS loading buffer was added to stop the reaction, and the changes in the content of SuSy3-His protein were detected by immunoblotting using His antibody. It was found that the protein content of SuSy3 protein in the extract of wild-type fruits is gradually decreased with the passage of time; while in the mutant cdpk8-1, the rate of protein degradation is significantly slowed down, indicating that phosphorylation of SuSy3 by CDPK8 promotes its protein degradation (Fig. 5B).

### Step 4: In vitro phosphorylation experiments proved that CDPK8 can phosphorylate Serine 11 of SuSy3 protein

Fig. 6 shows an in vitro phosphorylation assay proving that CDPK8 can phosphorylate the serine at position 11 (S11) of the SuSy3 protein. When the serine at position 11 mutates to alanine (SlSuSy3^{S11A}), there is no autoradiographic band, indicating that CDPK8 cannot phosphorylate SlSuSy3^{S11A}, S11 is the modification site for CDPK8 to phosphorylate SuSy3.

### Example 5: Specific Overexpression of SuSy3 Gene in Fruits by Transgenic Technology

*Agrobacterium-mediated* transgenic technology is used to specifically over-express the SuSy3 gene in fruits, thereby obtaining transgenic plants in which the SuSy3 gene is specifically over-expressed in fruits. The specific steps are as follows.

### Step 1: cloning SuSy3 genomic sequence

Primers were designed on the genome of SuSy3 gene, and the SuSy3 genomic sequence was cloned.

### Step 2: Construction of E8-SuSy3 overexpression vector

The original vector contains the E8 promoter sequence, which is a tomato fruit-specific expression promoter. The SuSy3 genomic sequence cloned in step 1 was further inserted into the vector to obtain the E8-SuSy3 overexpression vector.

### Step 3: Obtaining transgenic plants

The E8-SuSy3 overexpression vector obtained in step 2 was transferred to *Agrobacterium* competent cells EHA105 by heat shock transformation (*Agrobacterium* EHA105 competent cells were purchased from Shanghai Weidi Biotechnology Co., Ltd., and are available to the public) to obtain the recombinant bacteria EHA105/E8-SuSy3.

The recombinant bacteria EHA105/E8-SuSy3 was then transformed by *Agrobacterium-*infected tomato transformation method (the recombinant *Agrobacterium* was propagated at 28°C, and the propagated bacterial solution was used to infect tomatoes). After kanamycin resistance screening, the T₀ generation transgenic tomato plants were obtained.

### Step 4: Identification of transgenic plants overexpressing SuSy3 gene

The leaves of the T₀ generation transgenic tomato plants obtained in step 3 of Example 5 were collected, and genomic DNA was extracted as a template. PCR amplification was performed by using the following primer pairs, then detecting the PCR amplification products of different strains.

The sequences of the primers for detecting overexpression of the SuSy3 gene are as follows.

E8-test-F: ATGGCATCCTCATATTGAGAT (SEQ ID NO: 20).

SuSy3-OE-R: TATTCCTCGGCCTTCCTCAG (SEQ ID NO: 21).

Using the leaves of non-transgenic tomato plants as the control, the PCR amplification products of different strains were analyzed by agarose gel electrophoresis. The control leaves should have no bands, while some T0 generation transgenic tomato plant leaves had bands. The strains with bands were the T0 generation SuSy3 gene overexpression transgenic strains.

The SuSy3 gene overexpression transgenic line T0 identified in this case was used for the identification of tomato SSC and fruit weight phenotypes as described below.

### Example 6: Phenotypic Identification of SuSy3 Overexpression Transgenic Lines and Fruit-Specifically Expressing Transgenic Lines

The same method as in Example 5 was used to prepare a SuSy3 overexpression transgenic lines (35S-SuSy3-1/2/3).

The SuSy3 overexpression transgenic lines (35S-SuSy3-1/2/3) and the fruit-specific expression transgenic lines (E8-SuSy3-1/2/3) obtained in Example 5 were subjected to SSC and fruit weight phenotype identification.

Fig. 7 shows the results of phenotypic identification of SuSy3 overexpression transgenic lines (35S-SuSy3-1/2/3) and fruit-specific expression transgenic lines (E8-SuSy3-1/2/3): overexpression of SuSy3 can significantly increase the SSC (Fig. 7B), and the SSC is inversely proportional to the fruit weight (Fig. 7A); specific expression of SuSy3 in fruits can significantly increase the SSC (Fig. 7D) and has no effect on the fruit weight (Fig. 7C).

The above are only preferred embodiments of the present disclosure. It should be pointed out that for a person skilled in the art, several improvements and modifications can be made without departing from the principle of the present disclosure. These improvements and modifications should also be regarded as falling in the protection scope of the present disclosure.

## Claims

1. A polynucleotide, **characterized by** comprising at least one of the following nucleotide sequences:
(11) a sequence as set forth in any one of SEQ ID NOs: 3 and 23-36;
(12) a complementary sequence, degenerate sequence or homologous sequence of any one of the sequences set forth in SEQ ID NOs: 3 and 23-36, where the homologous sequence is a sequence having 75% or more identity to any one of the sequences set forth in SEQ ID NOs: 3 and 23-36;
(13) a sequence that hybridizes to any of the sequences set forth in SEQ ID NOs: 3 and 23-36 under stringent conditions, or a complementary sequence thereof;
(14) a cDNA sequence of any one of the sequences described in the above items (11) to (13);
(15) a sequence as set forth in any one of SEQ ID NOs: 11 and 95-108;
(16) a complementary sequence, degenerate sequence or homologous sequence of any one of the sequences set forth in SEQ ID NOs: 11, and 95-108, where the homologous sequence is a sequence having 75% or more identity to any one of the sequences set forth in SEQ ID NOs: 11 and 95-108;
(17) a sequence that hybridizes to any one of the sequences set forth in SEQ ID NOs: 11 and 95-108 under stringent conditions, or a complementary sequence thereof; and
(18) a cDNA sequence of any one of the sequences described in the above items (15) to (17).

2. A protein, **characterized in that** it is at least one of the following sequences:
(21) a sequence as set forth in any one of SEQ ID NOs: 2 and 66-94;
(22) a fusion protein obtained by connecting a tag to N-terminus and/or C-terminus of any of the sequences set forth in SEQ ID NOs: 2 and 66-94;
(23) a protein having the same function obtained by substituting and/or deleting and/or adding one or more amino acid residues in any of the sequences set forth in SEQ ID NOs: 2 and 66-94;
(24) a protein having 75% or more identity to any one of the sequences set forth in SEQ ID NOs: 2 and 66-94 and having the same function;
(25) any one of the sequences set forth in SEQ ID NOs: 10 and 153-196;
(26) a fusion protein obtained by connecting a tag to the N-terminus and/or C-terminus of any of the sequences set forth in SEQ ID NOs: 10 and 153-196;
(27) a protein having the same function obtained by substituting and/or deleting and/or adding one or more amino acid residues in any of the sequences set forth in SEQ ID NOs: 10 and 153-196; and
(28) a protein having 75% or more identity with any one of the sequences set forth in SEQ ID NOs: 10 and 153-196 and having the same function.

3. A biomaterial, **characterized in that** it is any one of the following (31) to (35):
(31) a knockout cassette for knocking out the polynucleotide of claim 1;
(32) a knockout vector for knocking out the polynucleotide of claim 1;
(33) a recombinant microorganism for knocking out the polynucleotide of claim 1;
(34) a plant cell line for knocking out the polynucleotide of claim 1; and
(35) a plant protoplast, cell or callus tissue in which any one of the above items (31) to (33) is introduced.

4. Use of any one of the polynucleotide of claim 1, the protein of claim 2, and the biological material of claim 3 in the improvement of the quality of plant fruits.

5. The use of claim 4, **characterized in that** the plant is a berry plant.

6. The use of claim 4 or 5, **characterized in that** the improvement of the quality of plant fruits comprises: increasing the content of soluble solids, sugar, and citric acid in the plant fruits, reducing the content of malic acid, and maintaining the weight of the fruits;
preferably, the sugar comprises one or more of glucose, fructose and sucrose.

7. Use of a polynucleotide, protein and biological material in the improvement of a quality of a plant fruit, **characterized in that** the phosphorylation level of SuSy3 protein is reduced by at least one of the following methods:
(1) inhibiting an expression of CDPK or inactivating a phosphorylase of CDPK; a polynucleotide of CDPK gene is the polynucleotide of claim 1, or CDPK protein is the protein of claim 2;
(2) mutating one or more amino acids in SuSy3 protein, wherein a mutation site is a phosphorylation site mediated by CDPK, without altering the glycosyltransferase activity of the SuSy3 protein;
(3) specifically overexpressing the SuSy3 protein in the fruit.

8. The use of claim 7, **characterized in that** in the above method (1), the method for inhibiting the expression of CDPK or inactivating the phosphorylase of CDPK comprises regulating a promoter and/or changing a coding region.

9. The use of claim 7 or 8, **characterized in that** in the above method (2), the mutation site is serine at position 11 of the SuSy3 protein.

10. The use of any one of claims 7 to 9, **characterized in that** in the above method (3), the promoter for specifically overexpressing the SuSy3 protein in the fruit is E8 promoter.

11. A plant or a related product thereof, **characterized in that** the plant is one of the following plants:
(1) a plant obtained by using the biological material of claim 3, or a plant obtained by using the method described in any one of claims 7 to 10;
(2) a progeny formed by self-pollination of the plant described in the above item (1), and a plant grown from the progeny; and
(3) a progeny formed by hybridization of the plant described in the above item (1) with other varieties, and a plant grown from the progeny.

12. The plant or a related product thereof of claim 11, **characterized in that** the related product is a plant part, plant cell, tissue culture, protoplast, fruit, pollen or seed.

13. A commercial plant product, **characterized in that** it is a commercial plant product made from the plant of claim 11 or 12 or a related product thereof; the commercial plant product comprises food, medicine, cosmetics/skin care product, feed or other products.

14. The commercial plant product of claim 13, **characterized in that** the food comprises: fresh fruit, dried fruit, frozen fruit, pickled fruit, candied fruit, fried fruit or various processed forms of fruit; the various processed forms of fruit comprise fruit strip, fruit block, fruit slice, fruit particle, fruit powder, fruit paste, fruit juice, or fermented product;
the medicine comprises: various dosage forms made from medicinal parts of the plant;
the cosmetics/skin care product comprises: facial cleanser, toner, lotion, face cream, essence, facial mask, eye cream, eye mask, primer lotion, sunscreen, liquid foundation, or BB cream; and
the feed comprises: liquid feed, solid feed, semi-solid feed, or feed raw material.

15. A method for producing a commercial plant product, **characterized by** comprising obtaining the plant or a related product thereof of claim 11 or 12 to produce the commercial plant product.

16. An improved tomato plant or seed, **characterized by** comprising one or more genetic modifications and is capable of producing tomato fruits at maturity, and it comprises:
(i) an average total soluble solids content (SSC) which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
wherein both the control tomato plant or seed and the modified tomato plant or seed comprise substantially the same genetic background, except for the one or more genetic modifications.

17. An improved tomato plant or seed, **characterized by** comprising one or more genetic modifications and is capable of producing tomato fruits at maturity, and it comprises:
(i) an average glucose level which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
wherein both the control tomato plant or seed and the modified tomato plant or seed comprise substantially the same genetic background, except for the one or more genetic modifications.

18. An improved tomato plant or seed, **characterized by** comprising one or more genetic modifications and is capable of producing tomato fruits at maturity, and it comprises:
(i) an average fructose level which is at least 5% higher than that of a control tomato plant or seed, preferably at least 8%, 10%, 12%, 15%, 18%, 20%, 22%, 25%, 28%, 30%, 32%, 35%, 38% or 40% higher; and
(ii) an average fresh fruit weight substantially similar to that of a control tomato plant and seed;
wherein both the control tomato plant or seed and the modified tomato plant or seed comprise substantially the same genetic background, except for the one or more genetic modifications.

19. The improved tomato plant or seed of any one of claims 16 to 18, **characterized in that** the one or more genetic modifications are located in a gene selected from the group consisting of: CDPK8, CDPK9, SuSy3, and a homolog thereof.

20. A breeding method for improving plant fruit quality, **characterized by** comprising using a molecular marker to assist in plant breeding, wherein the molecular marker is a molecular marker for detecting presence or absence of CDPK8 and/or CDPK9.
